(19) Europäisches Patentamt / European Patent Office / Office européen des brevets

(11) EP 4 101 521 A1

(12) # EUROPEAN PATENT APPLICATION
published in accordance with Art. 153(4) EPC

(43) Date of publication:
**14.12.2022 Bulletin 2022/50**

(21) Application number: **21750274.9**

(22) Date of filing: **26.01.2021**

(51) International Patent Classification (IPC):
***B01D 61/36*** (2006.01) ***B01D 69/00*** (2006.01)
***B01D 69/02*** (2006.01) ***B01D 71/06*** (2006.01)
***B01D 71/64*** (2006.01)

(52) Cooperative Patent Classification (CPC):
**B01D 61/36; B01D 69/00; B01D 69/02; B01D 71/06; B01D 71/64**

(86) International application number:
**PCT/JP2021/002638**

(87) International publication number:
**WO 2021/157433 (12.08.2021 Gazette 2021/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.02.2020 JP 2020018651**

(71) Applicant: **NITTO DENKO CORPORATION**
**Ibaraki-shi**
**Osaka 567-8680 (JP)**

(72) Inventors:
- **SHIMAZU Akira**
**Ibaraki-shi, Osaka 567-8680 (JP)**
- **KATAGIRI Makoto**
**Ibaraki-shi, Osaka 567-8680 (JP)**
- **SUGITANI Tooru**
**Ibaraki-shi, Osaka 567-8680 (JP)**
- **NISHIYAMA Shinya**
**Ibaraki-shi, Osaka 567-8680 (JP)**
- **MAEDA Kazuhisa**
**Ibaraki-shi, Osaka 567-8680 (JP)**

(74) Representative: **Hoffmann Eitle**
**Patent- und Rechtsanwälte PartmbB**
**Arabellastraße 30**
**81925 München (DE)**

(54) **SEPARATION MEMBRANE AND METAL ORGANIC STRUCTURE**

(57) The present invention provides a separation membrane suitable for separating water from a liquid mixture containing an alcohol and water. The separation membrane of the present invention includes a metal organic framework. On the separation membrane, at least one of requirements (i) and (ii) below holds: (i) a ratio R1 of a number N2 of molecules satisfying a specified condition (b) with respect to a number N1 of molecules satisfying a specified condition (a) is less than 0.29; (ii) a ratio R2 of an adsorption amount of water adsorbed by the metal organic framework under water vapor at 25°C and 3.2 kPa with respect to an adsorption amount of ethanol adsorbed by the metal organic framework under an ethanol atmosphere at 25°C and 7.4 kPa is more than 4.0.

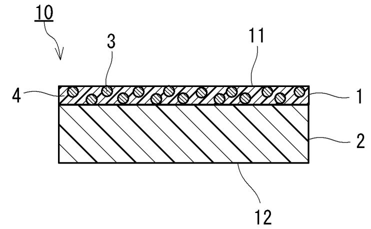


FIG.1

EP 4 101 521 A1

## Description

### TECHNICAL FIELD

The present invention relates to a separation membrane suitable for separating water from a liquid mixture containing an alcohol and water, and a metal organic framework.

### BACKGROUND ART

A pervaporation method and a vapor permeation method have been developed as methods for separating water from a liquid mixture containing an alcohol and water. These methods are particularly suitable for separating water from an azeotropic mixture such as a liquid mixture containing ethanol and water. The pervaporation method is also characterized in that it does not require the liquid mixture to be evaporated before being treated.

Examples of a material of a separation membrane used in the pervaporation method include zeolite, polyvinyl alcohol (PVA), and polyimide. Zeolite and PVA have a high hydrophilicity. Thus, when a content of the water in the liquid mixture is high, the separation membrane made of zeolite or PVA swells with the water, lowering the separation performance of the separation membrane in some cases.

In contrast, polyimide is a material that can better suppress the swelling with water than zeolite and PVA. For example, Patent Literature 1 discloses to use an asymmetric membrane made of polyimide as a separation membrane for the pervaporation method.

### CITATION LIST

#### Patent Literature

Patent Literature 1: JP 2013-528118 A

### SUMMARY OF INVENTION

#### Technical Problem

In the case of separating water from a liquid mixture containing an alcohol and water by using a conventional separation membrane, a flux of the water permeating through the separation membrane is left to be enhanced. From a first viewpoint above, in the case of separating water from a liquid mixture containing an alcohol and water, a separation membrane suitable for increasing the flux of the water permeating therethrough is desired.

In the case of separating water from a liquid mixture containing an alcohol and water by using a conventional separation membrane, the separation performance of the membrane is left to be enhanced. From a second viewpoint above, in the case of separating water from a liquid mixture containing an alcohol and water, a separation membrane that exhibits high separation performance is desired.

Therefore, the present invention is intended to provide, from the viewpoints mentioned above, a separation membrane suitable for separating water from a liquid mixture containing an alcohol and water.

#### Solution to Problem

Taking the first viewpoint into consideration, the present invention provides, from a first aspect, a separation membrane including a metal organic framework, wherein

at least one of requirements (i) and (ii) below holds:

(i) when the metal organic framework satisfies a condition (a) in which, when a number N1 of water molecules is/are inserted inside the metal organic framework, a potential energy of a composite composed of the metal organic framework and the water molecule(s) is lower than a total value of a potential energy of the metal organic framework in an isolated state before the water molecule(s) is/are inserted and a potential energy of the water molecule(s) in an isolated state, and a difference between the potential energy of the composite and the total value is maximum, and a condition (b) in which, when a number N2 of ethanol molecules is/are inserted inside the metal organic framework, a value obtained by subtracting a total value of a potential energy of the metal organic framework in an isolated state before the ethanol molecule(s) is/are inserted and a potential energy of the ethanol molecule(s) in an isolated state from a potential energy of a composite composed of the metal

organic framework and the ethanol molecule(s) is minimum,
a ratio R1 of the number N2 with respect to the number N1 is less than 0.29;
(ii) a ratio R2 of an adsorption amount of water adsorbed by the metal organic framework under water vapor at 25°C and 3.2 kPa with respect to an adsorption amount of ethanol adsorbed by the metal organic framework under an ethanol atmosphere at 25°C and 7.4 kPa is more than 4.0.

Taking the second viewpoint into consideration, the present invention provides, from a second aspect, a separation membrane including a metal organic framework, wherein a crystal structure of the metal organic framework has a volume change ratio, calculated by a molecular simulation, of 0% or less with respect to ethanol.

Furthermore, the present invention provides, from another aspect, a metal organic framework used for separating water from a liquid mixture containing an alcohol and water, wherein

at least one of requirements (i) and (ii) below holds:

(i) when the metal organic framework satisfies a condition (a) in which, when a number N1 of water molecules is/are inserted inside the metal organic framework, a potential energy of a composite composed of the metal organic framework and the water molecule(s) is lower than a total value of a potential energy of the metal organic framework in an isolated state before the water molecule(s) is/are inserted and a potential energy of the water molecule(s) in an isolated state, and a difference between the potential energy of the composite and the total value is maximum, and a condition (b) in which, when a number N2 of ethanol molecules is/are inserted inside the metal organic framework, a value obtained by subtracting a total value of a potential energy of the metal organic framework in an isolated state before the ethanol molecule(s) is/are inserted and a potential energy of the ethanol molecule(s) in an isolated state from a potential energy of a composite composed of the metal organic framework and the ethanol molecule(s) is minimum,

a ratio R1 of the number N2 with respect to the number N1 is less than 0.29;
(ii) a ratio R2 of an adsorption amount of water adsorbed by the metal organic framework under water vapor at 25°C and 3.2 kPa with respect to an adsorption amount of ethanol adsorbed by the metal organic framework under an ethanol atmosphere at 25°C and 7.4 kPa is more than 4.0.

Advantageous Effects of Invention

In separating water from a liquid mixture containing an alcohol and water, the separation membrane provided from the first aspect of the present invention is suitable for increasing a flux of the water permeating therethrough.

In separating water from a liquid mixture containing an alcohol and water, the separation membrane provided from the second aspect of the present invention tends to exhibit high separation performance.

BRIEF DESCRIPTION OF DRAWINGS

FIG. 1 is a cross-sectional view illustrating schematically a separation membrane according one embodiment of the present invention.
FIG. 2 is a graph for explaining a method for determining a number N1 of molecules.
FIG. 3 is a schematic cross-sectional view of a membrane separation device provided with the separation membrane of the present invention.
FIG. 4 is a perspective view illustrating schematically a modification of the membrane separation device provided with the separation membrane of the present invention.

DESCRIPTION OF EMBODIMENTS

According to one embodiment of the present invention, the separation membrane is used for separating water from a liquid mixture containing an alcohol and water.

According to one embodiment of the present invention, at least one of requirements that the ratio R1 is 0.14 or less and that the ratio R2 is 7.0 or more holds.

According to one embodiment of the present invention, the metal organic framework has a void fraction of 40% or more.

According to one embodiment of the present invention, the metal organic framework has a largest cavity

diameter of 0.3 nm or more.

According to one embodiment of the present invention, the metal organic framework includes at least one selected from the group consisting of MAF-Mg, TEVZIF, and JEDKIM.

According to one embodiment of the present invention, a crystal structure of the metal organic framework has a volume change ratio, calculated by a molecular simulation, of 0% or less with respect to ethanol.

According to one embodiment of the present invention, a crystal structure of the metal organic framework has a volume change ratio, calculated by a molecular simulation, of 0% or more with respect to water.

According to one embodiment of the present invention, the separation membrane includes a matrix containing polyimide, and the metal organic framework is dispersed in the matrix.

According to one embodiment of the present invention, the above-mentioned polyimide includes a structural unit represented by formula (1) below:

[Chemical Formula 1]

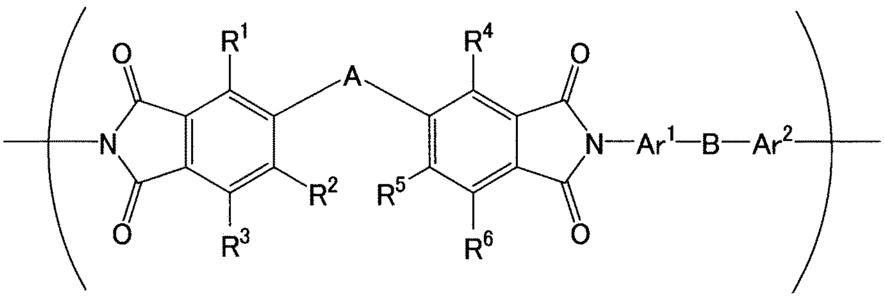

(1)

where A is a linking group having a solubility parameter, in accordance with a Fedors method, of more than 5.0 $(cal/cm^3)^{1/2}$; B is a linking group having a solubility parameter, in accordance with the Fedors method, of more than 8.56 $(cal/cm^3)^{1/2}$; $R^1$ to $R^6$ each are independently a hydrogen atom, a halogen atom, a hydroxyl group, a sulfonic group, an alkoxy group having 1 to 30 carbon atoms, or a hydrocarbon group having 1 to 30 carbon atoms; $Ar^1$ and $Ar^2$ each are a divalent aromatic group; and $Ar^1$ and $Ar^2$ each are represented by formula (2) below when $Ar^1$ and $Ar^2$ each are a phenylene group that may have a substituent;

[Chemical Formula 2]

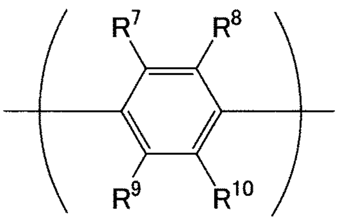

(2)

where $R^7$ to $R^{10}$ each are independently a hydrogen atom, a halogen atom, a hydroxyl group, a sulfonic group, an alkoxy group having 1 to 30 carbon atoms, or a hydrocarbon group having 1 to 30 carbon atoms.

According to one embodiment of the present invention, when, in a state in which a liquid mixture composed of ethanol and water is in contact with one surface of the separation membrane, a space adjacent to an other surface of the separation membrane is decompressed, a flux of the water permeating through the separation membrane is 0.12 $kg/m^2/hr$ or more. A concentration of the ethanol in the liquid mixture is 50 vol% when measured with a temperature of the liquid mixture at 20°C, the liquid mixture in contact with the separation membrane has a temperature of 60°C, and the space is decompressed in such a manner that a pressure in the space is lower than an atmospheric pressure in a measurement environment by 100 kPa.

According to one embodiment of the present invention, the separation membrane has a separation factor $\alpha$ of 20 or more for water with respect to ethanol. In a state in which a liquid mixture composed of ethanol and water is in contact with one surface of the separation membrane, the separation factor $\alpha$ is measured by decompressing a space adjacent to an other surface of the separation membrane. A concentration of the ethanol in the liquid mixture is 50 vol% when measured with a temperature of the liquid mixture at 20°C, the liquid mixture in contact with the separation

membrane has a temperature of 60°C, and the space is decompressed in such a manner that a pressure in the space is lower than an atmospheric pressure in a measurement environment by 100 kPa.

Hereinafter, embodiments of the present invention will be described. The following description is not intended to limit the present invention to specific embodiments. Basically, repetitive descriptions in these embodiments are omitted. To each of the embodiments, descriptions about the other embodiment can be applied except when those descriptions are obviously inapplicable thereto.

(Embodiment 1)

First, one embodiment of the separation membrane provided from the first aspect will be described. As shown in FIG. 1, a separation membrane 10 of the present embodiment is provided with a separation functional layer 1. The separation functional layer 1 allows water contained in a liquid mixture to permeate therethrough preferentially or selectively. The separation membrane 10 may be further provided with a porous support member 2 supporting the separation functional layer 1.

The separation functional layer 1 has a filler 3 and a matrix 4, for example. The filler 3 is dispersed in the matrix 4 and is buried in the matrix 4. In the embodiment shown in FIG. 1, all particles of the filler 3 are spaced apart from each other. The filler 3 may be condensed partially.

The filler 3 includes a metal organic framework (MOF) S1. The metal organic framework is also referred to as a porous coordination polymer (PCP), and can take a low-molecular compound into itself. The metal organic framework S1 included in the filler 3 can take water molecules into itself more preferentially or selectively than it does alcohol molecules.

In the separation membrane 10 of the present embodiment, at least one of requirements (i) and (ii) below holds:

(i) when the metal organic framework S1 satisfies a condition (a) in which, when a number N1 of water molecules is/are inserted inside the metal organic framework S1, a potential energy $E_{MOF + H2O}$ of a composite composed of the metal organic framework S1 and the water molecule(s) is lower than a total value of a potential energy $E_{MOF}$ of the metal organic framework S1 in an isolated state before the water molecule(s) is/are inserted and a potential energy $E_{H2O}$ of the water molecule(s) in an isolated state, and a difference $|\Delta E1|$ between the potential energy $E_{MOF + H2O}$ of the composite and the total value is maximum, and a condition (b) in which, when a number N2 of ethanol molecules is/are inserted inside the metal organic framework S1, a value $\Delta E2$ obtained by subtracting a total value of a potential energy $E_{MOF}$ of the metal organic framework in an isolated state before the ethanol molecule(s) is/are inserted and a potential energy $E_{EtOH}$ of the ethanol molecule(s) in an isolated state from a potential energy $E_{MOF + EtOH}$ of a composite composed of the metal organic framework and the ethanol molecule(s) is minimum,

a ratio R1 of the number N2 with respect to the number N1 is less than 0.29;

(ii) a ratio R2 of an adsorption amount of water adsorbed by the metal organic framework S1 under water vapor at 25°C and 3.2 kPa with respect to an adsorption amount of ethanol adsorbed by the metal organic framework S1 under an ethanol atmosphere at 25°C and 7.4 kPa is more than 4.0.

First, the requirement (i) mentioned above will be described. The ratio R1 is preferably 0.27 or less, and it may be 0.25 or less, 0.20 or less, 0.17 or less, 0.14 or less, or 0.11 or less in some the cases. The lower limit of the ratio R1 is not particularly limited, and it is 0.01, for example.

The above-mentioned numbers N1 and N2 each are a value determined based on a plurality of factors such as a structure of the metal organic framework S1 and a chemical interaction between the metal organic framework S1 and a molecule(s) inserted inside the metal organic framework S1. The numbers N1 and N2 can be determined using a molecular simulation, for example. The molecular simulation can be carried out using a known software such as Materials Studio available from Dassault Systemes and Gaussian09 available from Gaussian, Inc., for example.

Hereinafter, an example of a method for determining the number N1 will be described. First, a crystal structure data of the metal organic framework S1 is acquired. The crystal structure data of the metal organic framework S1 may be acquired by measuring the metal organic framework S1 using a known method such as an X-ray diffraction method (XRD), or may be acquired from papers and known databases (such as Cambridge Structural Database (CSD)). When a crystal structure recorded on the crystal structure data includes a solvent, such as water, the solvent is removed from the crystal structure by processing the crystal structure data without changing a lattice constant of the crystal structure. Here, it is unnecessary to remove ionic species included in the crystal structure. Next, a unit cell of the crystal structure is extended to a supercell by processing the crystal structure data. Thereby, a crystal structure model is obtained. The crystal structure model has a cube shape with a side of 2 to 4 nm, for example. Then, a parameter as to force constant and a parameter as to point charge are assigned to all atoms constituting the crystal structure model. The parameter as to force constant is calculated by, for example, using a universal force field (UFF) as a force field. The parameter as

to point charge is calculated by, for example, using a charge equilibration (QEq) method. The crystal structure model can be created by, for example, using Materials Studio available from Dassault Systemes.

Next, a potential energy of the crystal structure model is calculated based on a molecular force field method. For the calculation of the potential energy, the universal force field (UFF) and the charge equilibration (QEq) method mentioned above are used, for example. The obtained potential energy can be assumed as the potential energy $E_{MOF}$ of the metal organic framework S1 in an isolated state. In the present description, the "isolated state" means a state in which a compound referred to is free from an interaction with its circumference.

Next, a molecular model of water is created. The molecular model of water can be created by, for example, using a structural optimization calculation by a density functional method. The structural optimization calculation can be made by, for example, using Gaussian09 available from Gaussian, Inc. In this case, B3LYP may be used as a functional and 6-31G(d) may be used as a basis function. Subsequently, a parameter as to point charge derived from an electrostatic potential is assigned to all atoms constituting the molecular model. The parameter as to point charge can be calculated by an MK (Merz-Kollman) method, for example. Next, a potential energy of the molecular model of water is calculated based on a molecular force field method. For this calculation of the potential energy, the above-mentioned universal force field (UFF) is used, for example. First, a potential energy of water per molecule is calculated. A potential energy of two or more water molecules is calculated by multiplying the potential energy of water per molecule by the number of the water molecules. The potential energy obtained by such a method can be assumed as the potential energy $E_{H2O}$ of the water molecule(s) in an isolated state.

Next, a complex model in which the molecular model of water is inserted inside the crystal structure model of the metal organic framework S1 is created. As stated herein, a plurality of the complex models in which the molecular model(s) is/are inserted in different numbers, respectively, are created. A position at which each of the molecular model(s) is inserted is determined using a Monte Carlo method, for example. In each of the complex models, it can be assumed that the molecular model(s) of water is/are adsorbed (sorbed) to an inside of the metal organic framework S1. Next, a potential energy of each of the complex models is calculated by making a structural optimization calculation without changing the structure of the metal organic framework S1. For the structural optimization calculation, the UFF is used as a force field, for example. The obtained potential energy of each of the complex models can be assumed as the potential energy $E_{MOF + H2O}$ of the composite composed of the metal organic framework S1 and the water molecule(s). In the present description, the "composite" means a complex in which a molecule(s) is/are inserted inside the metal organic framework.

Next, the value ΔE1 is calculated by subtracting the total value of the potential energy $E_{MOF}$ of the metal organic framework S1 and the potential energy $E_{H2O}$ of the water molecule(s) from the potential energy $E_{MOF + H2O}$ of the composite. Specifically, the value ΔE1 can be calculated by formula (I) below. $E_{H2O}$ in the formula (I) means a potential energy of the same number of water molecules in an isolated state as the number of the water molecules included in the composite. ΔE1 represents a potential energy change caused by the insertion of the water molecule(s).

$$\Delta E1 = E_{MOF + H2O} - (E_{MOF} + E_{H2O}) \quad (I)$$

Next, a graph showing a relationship between a number N of the molecular models of water (a number N of the water molecules) inserted inside the metal organic framework S1 and the value ΔE1 is created based on the obtained result. FIG. 2 shows an example of the graph. As shown in FIG. 2, in the metal organic framework S1 of the present embodiment, the value ΔE1 tends to decrease as the number N of the molecular models of water increases until the number N of the molecular models of water reaches a specific value. In contrast, when the number N of the molecular models of water exceeds the specific value, the value ΔE1 tends to increase as the number N of the molecular models of water increases. Next, a range in which the value ΔE1 is a negative value is determined from this graph. That the value ΔE1 is a negative value means the potential energy $E_{MOF + H2O}$ of the composite is less than the total value of the potential energy $E_{MOF}$ of the metal organic framework S1 and the potential energy $E_{H2O}$ of the water molecule(s). Next, the number N of the molecular models of water when the value ΔE1 is minimum (smallest), that is, when the value |ΔE1| is maximum, in the range in which the value ΔE1 is a negative value is determined. The value |ΔE1| is equivalent to a difference between the potential energy $E_{MOF + H2O}$ of the composite and the total value of the potential energy $E_{MOF}$ of the metal organic framework S1 and the potential energy $E_{H2O}$ of the water molecule(s). In FIG. 2, in the range in which the value ΔE1 is a negative value, the number N of the molecular models of water when the value |ΔE1| is maximum is 145. The number N of the molecular models of water determined by the above-mentioned method can be assumed as the number N1.

That the value ΔE1 is a negative value means that an energy is released out of the composite, that is, heat is generated, by the insertion of the water molecule(s) inside the metal organic framework S1. In the range in which the value ΔE1 is a negative value, the composite is in a most stable state when the value |ΔE1| is maximum. It should be noted that a metal organic framework with the value ΔE1 that fails to be a negative value (with the value ΔE1 that is

always a positive value) is not suitable for the separation membrane of the present embodiment.

The minimum value of $\Delta$E1 is not particularly limited. For example, the minimum value of $\Delta$E1 may be -7400 kcal/mol to -1700kcal/mol when the crystal structure model has a molecular weight of 22000 to 94000. The number N1 is not particularly limited, either. For example, the number N1 may be 300 to 870 when the crystal structure model has a molecular weight of 22000 to 94000.

Next, an example of a method for determining the number N2 will be described. First, the crystal structure model of the metal organic framework S1 is created by the method mentioned above, and the potential energy $E_{MOF}$ of the metal organic framework S1 in an isolated state is determined.

Next, a molecular model of ethanol is created by the method mentioned above for the molecular model of water. A potential energy of the molecular model of ethanol is calculated based on a molecular force field method. For this calculation of the potential energy, the above-mentioned universal force field (UFF) is used, for example. First, a potential energy of ethanol per molecule is calculated. A potential energy of two or more ethanol molecules is calculated by multiplying the potential energy of ethanol per molecule by the number of the ethanol molecules. The potential energy obtained by such a method can be assumed as the potential energy $E_{EtOH}$ of the ethanol molecule(s) in an isolated state.

Next, a complex model in which the molecular model of ethanol is inserted inside the crystal structure model of the metal organic framework S1 is created by the above-mentioned method. As stated herein, a plurality of the complex models in which the molecular model(s) is/are inserted in different numbers, respectively, are created. Next, a potential energy of each of the complex models is calculated by the above-mentioned method. The obtained potential energy of each of the complex models can be assumed as the potential energy $E_{MOF + ETOH}$ of the composite composed of the metal organic framework S1 and the ethanol molecule(s).

Next, the value $\Delta$E2 is calculated by subtracting the total value of the potential energy $E_{MOF}$ of the metal organic framework S1 and the potential energy $E_{EtOH}$ of the ethanol molecule(s) from the potential energy $E_{MOF + EtOH}$ of the composite. Specifically, the value $\Delta$E2 can be calculated by formula (II) below. $E_{EtOH}$ in the formula (II) means a potential energy of the same number of ethanol molecules in an isolated state as the number of the ethanol molecules included in the composite. $\Delta$E2 represents a potential energy change caused by the insertion of the ethanol molecule(s).

$$\Delta E2 = E_{MOF + EtOH} - (E_{MOF} + E_{EtOH}) \qquad (II)$$

Next, a graph showing a relationship between a number N of the molecular models of ethanol (a number N of the ethanol molecules) inserted inside the metal organic framework S1 and the value $\Delta$E2 is created based on the obtained result. This graph tends to form a shape similar to that of a quadratic function as in FIG. 2. However, the graph may not form a shape similar to that of a quadratic function. For example, the value $\Delta$E2 may increase simply as the number N of the molecular models of ethanol increases. Next, the number N of the molecular models of ethanol when the value $\Delta$E2 is minimum is determined based on the graph. The number N of the molecular models of the ethanol determined by the above-mentioned method can be assumed as the number N2. The number N2 may be 0. For example, in the graph, when the value $\Delta$E2 increases simply as the number N of the molecular models increases, the number N (the number N2) of the molecular models of ethanol when the value $\Delta$E2 is minimum is considered to be 0.

The minimum value of $\Delta$E2 is not particularly limited. For example, the minimum value of $\Delta$E2 may be -2700 kcal/mol to -390 kcal/mol when the crystal structure model has a molecular weight of 22000 to 94000. The number N2 is not particularly limited, either. For example, the number N2 may be 30 to 230 when the crystal structure model has a molecular weight of 22000 to 94000.

Subsequently, the requirement (ii) mentioned above will be described. The ratio R2 is preferably 5.0 or more, and it may be 6.0 or more, 7.0 or more, 8.0 or more, 9.0 or more, or 10.0 or more in some cases. The upper limit of the ratio R2 is not particularly limited, and it is 30, for example. In the present description, the "adsorption amount" means a value obtained by converting a volume of a gas adsorbed by 1 g of the metal organic framework S1 into a volume of the gas in a standard state (298K, 1 atm).

An adsorption amount Q1 of ethanol adsorbed by the metal organic framework S1 can be determined by the following method. First, a filler composed of the metal organic framework S1 is prepared. This filler is pretreated by being heated under a decompressed atmosphere. The pretreatment may be carried out under a vacuum atmosphere. The pretreatment is carried out at a temperature of 100°C or higher, for example. The duration of the pretreatment is not particularly limited, and it is 1 hour or longer, for example. Next, the filler is placed in a known vapor adsorption amount measuring apparatus such as BELSORP-maxII available from MicrotracBEL Corp. Next, gaseous ethanol is introduced into the measuring apparatus at a measurement temperature of 25°C. The gaseous ethanol introduced is adsorbed by the filler. The gaseous ethanol is introduced until the pressure of the ethanol in the measuring apparatus reaches 7.4 kPa. The pressure of 7. 4 kPa is equivalent to an equilibrium vapor pressure (a saturation vapor pressure) of ethanol at 25°C. The adsorption of the ethanol by the filler is confirmed to have reached a state of equilibrium, and then the adsorption amount of the ethanol adsorbed by the filler is determined. The fact that the adsorption of the ethanol by the

filler has reached a state of equilibrium can be confirmed by a change in the pressure of the ethanol inside the measuring apparatus. For example, when the change in the pressure of the ethanol inside the measuring apparatus is 40 Pa or less for 500 seconds, the adsorption of the ethanol by the filler can be considered to have reached a state of equilibrium. The adsorption amount of the ethanol that is determined by the above-mentioned method can be assumed as the adsorption amount Q1.

An adsorption amount Q2 of water adsorbed by the metal organic framework S1 can be determined by the following method. First, a filler composed of the metal organic framework S1 is subject to the pretreatment mentioned above. This filler is placed in a vapor adsorption amount measuring apparatus. Next, water vapor is introduced into the measuring apparatus at a measurement temperature of 25°C. The water vapor is introduced until the pressure of the water vapor in the measuring apparatus reaches 3.2 kPa. The pressure of 3. 2 kPa is equivalent to an equilibrium vapor pressure of water at 25°C. The adsorption of the water by the filler is confirmed to have reached a state of equilibrium, and then the adsorption amount of the water adsorbed by the filler is determined. The determined adsorption amount of the water can be assumed as the adsorption amount Q2.

The adsorption amount Q1 of the ethanol adsorbed by the metal organic framework S1 is 50 $cm^3/g$ or less, for example. The lower limit of the adsorption amount Q1 is not particularly limited, and it is 12 $cm^3/g$, for example. The adsorption amount Q2 of the water adsorbed by the metal organic framework S1 is 110 $cm^3/g$ or more, for example. The upper limit of the adsorption amount Q2 is not particularly limited, and it is 610 $cm^3/g$, for example.

The metal organic framework S1 is not particularly limited as long as it satisfies at least one of the requirements (i) and (ii). The metal organic framework S1 has a void fraction of 35% or more, for example, and it is preferably 40% or more, and more preferably 43% or more. The upper limit of the void fraction of the metal organic framework S1 is not particularly limited, and it may be 70%, 60%, or 50%. The metal organic framework S1 has a largest cavity diameter of 0.3 nm or more, for example, and it is preferably 0.4 nm or more, and more preferably 0.45 nm or more. The upper limit of the largest cavity diameter of the metal organic framework S1 is not particularly limited, and it may be 2.0 nm or 1.5 nm. The void fraction and the largest cavity diameter of the metal organic framework S1 can be determined by analyzing the crystal structure data of the metal organic framework S1. The analysis of the crystal structure data of the metal organic framework S1 can be carried out using a known software such as Zeo++.

The metal organic framework S1 includes a metal ion and an organic ligand. Examples of the metal ion included in the metal organic framework S1 include an Mg ion, a Pb ion, and a Cu ion. The organic ligand may have a polar group. Examples of the polar group include a carboxyl group, a hydroxyl group, and an ester group. Examples of the organic ligand included in the metal organic framework S1 include: a carboxylic acid compound such as formic acid; polysaccharides such as cyclodextrin; and a lactone compound such as ascorbic acid.

Examples of the metal organic framework S1 include MAF-Mg (magnesium formate:$C_6H_6O_{12}Mg_3$),TEVZIF(catena-(bis($\mu_{17}$-γ-Cyclodextrin)-hexadeca-lead nonahydrate)), JEDKIM(catena-(bis($\mu_3$-Chloro)-hexakis($\mu_3$-1,2,3,4-tetrahydroxybutane-1,1-dicarboxylic acid-O,O,O',O",O‴,O"")-nona-copper(ii) hydrate)), MATCOB(catena-(tetrakis($\mu_2$-Quinoxaline)-tetra-copper($\mu_{12}$-phosphato)-($\mu_2$-hydroxo)-tetracosakis($\mu_2$-oxo)-decaoxo-di-copper-deca-tungsten sesquihydrate), QEGJAN(catena-(($\mu_2$-Dicyanamido)-aqua-(2,2'-bipyrimidine)-(dicyanamido)-manganese), DILKIU(catena-[tetrakis($\mu_3$-2-methylpropanoato)-bis($\mu_2$-2-formyl-4-methoxy-6-nitrophenoxy)-bis($\mu_2$-2-methylpropanoato)-tetra-manganese unknown solvate]), FELJAJ(catena-(($\mu_4$-2,2'-(1,4-Phenylenebis(carbamoylimino))bis(3-phenylpropanoato))-(dimethylformamide)-lead monohydrate)), BUSGUS(catena-(bis($\mu_2$-2-Amino-4-nitrobenzoato)-lead)), PITYUN01(catena-(($\mu_5$-3,5-bis(Trifluoromethyl)-1,2,4-triazolato)-bis($\mu_3$-3,5-bis(trifluoromethyl)-1,2,4-triazolato)-tri-silver(i))), HECRUE01(catena-(bis($\mu_2$-2,2-Dimethyl-1-(1-methyl-1H-pyrazol-4-yl)propan-1-one)-tetrakis(hexafluoroacetylacetonato)-di-copper heptane solvate)), CEHSUD(catena(Diaqua-($\mu_2$-L-tartrato)-manganese(ii) dihydrate)), MAJTOG(catena-(bis(Glycylglycylglycine)-hexacosaoxo-tetra-molybdenum nonahydrate)), HUZVED(catena-(Hexa-ammonium tetrakis($\mu_3$-hydroxo)-icosakis($\mu_3$-oxo)-tetrakis($\mu_2$-acetato-O,O')-decakis($\mu_2$-oxo)-hexa-aqua-docosaoxo-tri-calcium-octadeca-molybdenum(v,vi) tetradecahydrate)), UYEDIM(catena-[hexakis($\mu_3$-Oxo)-octakis($\mu_2$-oxo)-bis($\mu_2$-pyrazine-2-carboxylato-N1,O:N4)-tetra-aqua-deca-oxo-chromium-hexa-molybdenum-copper heptadecahydrate]), COVWAM(catena-(bis(4-Carboxypyridinium)($\mu_{12}$-silicato)-hexacosakis($\mu_2$-oxo)-triaqua-decaoxo-(4-pyridiniocarboxylic acid-O)-sodium-dodeca-tungsten(vi) hexahydrate)), ISIKIF(catena-(($\mu_7$-5,5'-(Ethane-1,2-diyl)bis(oxy)di-isophthalato)-triaqua-di-cobalt pentahydrate)), and FAKGOP(catena-(($\mu_7$-5,5'-(ethane-1,2-diylbis(oxy))diisophthalato)-triaqua-di-nickel(ii) pentahydrate)). The metal organic framework S1 includes, for example, at least one selected from the group consisting of MAF-Mg, TEVZIF, and JEDKIM.

The filler 3 includes the metal organic framework S1 as a main component, for example. In the present description, the "main component" means a component having a largest content in the filler 3 on weight basis. A content of the metal organic framework S1 in the filler 3 is 50 wt% or more, for example, and it is preferably 80 wt% or more, and more preferably 95% or more. The filler 3 is composed substantially of the metal organic framework S1, for example. The filler 3 may include another component other than the metal organic framework S1.

The filler 3 has a shape that is not particularly limited, and it is a particulate shape, for example. In the present description, the "particulate" is a shape such as a spherical shape, an elliptical shape, a flaky shape, and a fibrous shape.

The filler 3 has an average particle diameter that is not particularly limited, and it is 5 nm to 10000 nm, for example. The average particle diameter of the filler 3 can be determined by the following method, for example. A cross section of the separation functional layer 1 is observed with a transmission electron microscope. On an electron microscope image obtained, an area of a particular particle of the filler 3 is calculated by image processing. A diameter of a circle having an area equal to the calculated area is assumed as a diameter of that particular particle (a diameter of a particle) of the filler 3. A particle diameter of each of an arbitrary number (at least 50) of particles of the filler 3 is calculated. An average of the calculated values is assumed as the average particle diameter of the filler 3.

A content of the filler 3 in the separation functional layer 1 is 1wt% or more, for example, and it is preferably 3 wt% or more, more preferably 5 wt% or more, and still more preferably 8 wt% or more. The upper limit of the content of the filler 3 is not particularly limited, and it is 30 wt%, for example.

A material of the matrix 4 is not particularly limited. Examples of the material include zeolite, polyvinyl alcohol, and polyimide. Preferably, it is polyimide. As the polyimide contained in the matrix 4, polyimide (P) including a structural unit represented by formula (1) below can be mentioned, for example.

[Chemical Formula 3]

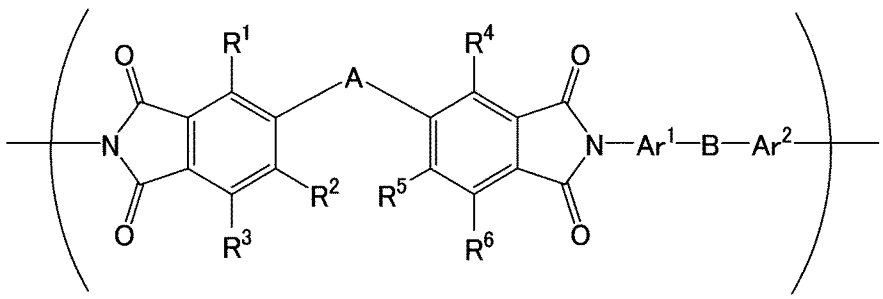

(1)

In the formula (1), A is, for example, a linking group having a solubility parameter, in accordance with a Fedors method, of more than 5.0 $(cal/cm^3)^{1/2}$. In the present description, the "solubility parameter in accordance with a Fedors method" is also referred to as an SP value. The "solubility parameter in accordance with a Fedors method" can be calculated by the following formula. It should be noted that in this formula, δi is the SP value of an atom or atomic group of an i component. Δei is an evaporation energy of the atom or atomic group of the i component. Δvi is a molar volume of the atom or atomic group of the i component.

$$\delta i[(cal/cm^3)^{1/2}] = (\Delta ei/\Delta vi)^{1/2}$$

The detail of the "solubility parameter in accordance with a Fedors method" is disclosed, for example, in "Polymer Engineering and Science" written by Robert F. Fedors, the year 1974, volume no. 14, the second issue, P. 147-154.

When the SP value of A is more than 5.0 $(cal/cm^3)^{1/2}$, water tends to penetrate into the separation functional layer 1 easily. The SP value of A is preferably 8.5 $(cal/cm^3)^{1/2}$ or more, more preferably 11.0 $(cal/cm^3)^{1/2}$ or more, and still more preferably 12.0 $(cal/cm^3)^{1/2}$ or more. The upper limit of the SP value of A is not particularly limited, and it may be 30.0 $(cal/cm^3)^{1/2}$, for example. Preferable examples of the SP value of A includes 12.0 $(cal/cm^3)^{1/2}$ and 12.68 $(cal/cm^3)^{1/2}$.

A includes, for example, at least one selected from the group consisting of an oxygen atom, a nitrogen atom, a sulfur atom, and a silicon atom. Preferably, A includes at least one selected from the group consisting of an oxygen atom and a nitrogen atom. Particularly preferably, A includes an oxygen atom. A includes, for example, at least one functional group selected from the group consisting of an ether group, an ester group, a ketone group, a hydroxyl group, an amide group, a thioether group, and a sulfonyl group. Preferably, A includes at least one selected from the group consisting of an ether group and an ester group.

A may include another group, such as a hydrocarbon group, besides the above-mentioned functional groups. The number of carbon atoms that the hydrocarbon group has is not particularly limited, and it is 1 to 15, for example. The number of carbon atoms may be 1 to 3, or may be 6 to 15. The valence of the hydrocarbon group is not particularly limited, either. Preferably, the hydrocarbon group is a divalent hydrocarbon group. Examples of the divalent hydrocarbon group include a methylene group, an ethylene group, a propane-1,3-diyl group, a propane-2,2-diyl group, a butane-1,4-diyl group, a pentane-1,5-diyl group, a 2,2-dimethylpropane-1,3-diyl group, a 1,4-phenylene group, a 2,5-di-tert-butyl-1,4-phenylene group, a 1-methyl-1,1-ethanediylbis(1,4-phenylene) group, and a biphenyl-4,4'-diyl group. Furthermore, at least one hydrogen atom included in these hydrocarbon groups may be substituted by a halogen atom.

A is a linking group represented, for example, by a general formula -O-$R^{19}$-O- or a general formula -COO-$R^{20}$-OOC-. As stated herein, $R^{19}$ and $R^{20}$ each are a divalent hydrocarbon group having 1 to 15 carbon atoms. As the divalent hydrocarbon group, the divalent hydrocarbon groups stated above can be mentioned.

A may not include the above-mentioned functional groups. Examples of such A include an alkylene group. The number of carbon atoms that the alkylene group has is not particularly limited, and it may be 1 to 15, for example, and it may be 1 to 5. The alkylene group may be branched, but preferably it is linear. A part of hydrogen atoms included in the alkylene group may be substituted by a halogen atom. However, it is preferable that the alkylene group be an alkylene group without the substitution, that is, a linear or branched alkylene group.

In the formula (1), the number of atoms constituting a bonding chain, among bonding chains that bond two phthalimide structures linked to each other by A, that is composed of a least number of atoms is 2 or more, for example, and it is preferably 4 or more, and more preferably 6 to 11. In the present description, the bonding chain composed of a least number of atoms is also referred to as a "shortest bonding chain". For example, in the case where A is an o-phenylene group, the number of atoms constituting a shortest bonding chain that bonds two phthalimide structures linked to each other by A is 2. In the case where A is a p-phenylene group, the number of atoms constituting a shortest bonding chain that bonds two phthalimide structures linked to each other by A is 4.

A may be one of the linking groups 1 to 26 shown in Tables 1 and 2 below. Tables 1 and 2 show the chemical structure, the SP value, and the number of atoms constituting a shortest bonding chain of each of the linking groups 1 to 26. A is preferably the linking group 11 or the linking group 18, and particularly preferably the linking group 18. In the case where A is the linking group 11 or the linking group 18, the polyimide (P) is easily dissolved in a polar organic solvent, such as N-methyl-2-pyrrolidone (NMP) and 1,3-dioxolane, and is easily adopted in a method desirable for manufacturing the separation functional layer 1.

[Table 1]

| | -A- | SP value [$(cal/cm^3)^{1/2}$] | The number of atoms constituting shortest bonding chain |
|---|---|---|---|
| 1 | $-CF_2-$ | 6.66 | 1 |
| 2 | $-CHC(CH_3)_3-$ | 7.52 | 1 |
| 3 | $-CH_2-$ | 8.56 | 1 |
| 4 | $-(CH_2)_5-$ | 8.56 | 5 |
| 5 | $-O-CH_2-C(CH_3)_2-CH_2-O-$ | 8.65 | 5 |
| 6 | $-O-(CH_2)_5-O-$ | 9.23 | 7 |
| 7 | $-O-(CH_2)_4-O-$ | 9.37 | 6 |
| 8 | 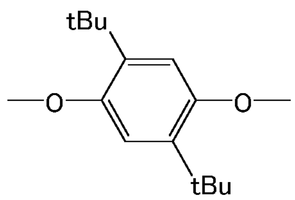 | 9.51 | 6 |
| 9 | 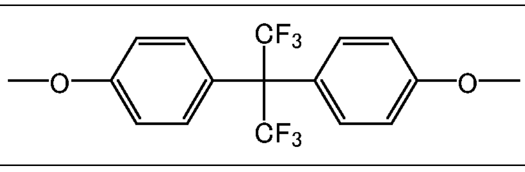 | 9.62 | 11 |
| 10 | $-O-CH_2-O-$ | 10.83 | 3 |
| 11 | | 11.02 | 11 |
| 12 | | 11.52 | 8 |
| 13 | | 12.00 | 7 |
| 14 | | 12.25 | 10 |

(continued)

| | -A- | SP value [(cal/cm$^3$)$^{1/2}$] | The number of atoms constituting shortest bonding chain |
|---|---|---|---|
| 15 | | 12.29 | 11 |

[Table 2]

| | -A- | SP value [(cal/cm$^3$)$^{1/2}$] | The number of atoms constituting shortest bonding chain |
|---|---|---|---|
| 16 | 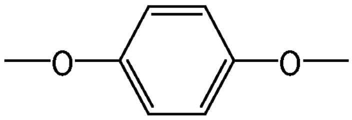 | 12.40 | 6 |
| 17 | $-SO_2-$ | 12.47 | 1 |
| 18 | 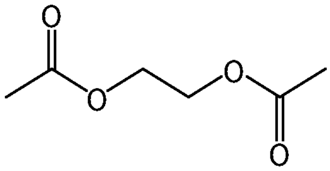 | 12.68 | 6 |
| 19 | 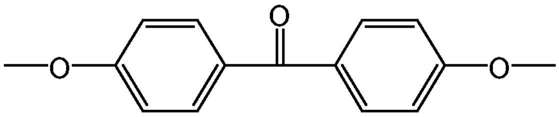 | 13.06 | 11 |
| 20 | 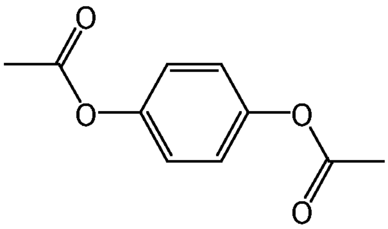 | 13.55 | 8 |
| 21 | -O- | 14.51 | 1 |
| 22 | -S- | 16.79 | 1 |
| 23 | 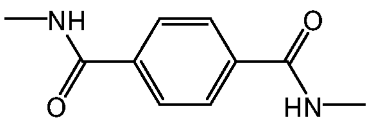 | 18.19 | 8 |
| 24 | -CO- | 19.60 | 1 |
| 25 | 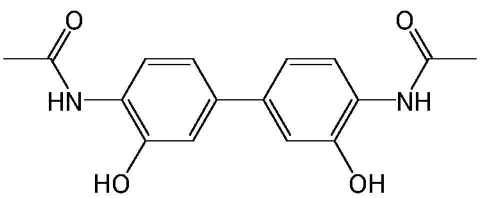 | 20.74 | 12 |
| 26 | -CONH- | 29.02 | 2 |

In the formula (1), B is, for example, a linking group having an SP value more than 8.56 (cal/cm$^3$)$^{1/2}$. When the SP value of the linking group B is more than 8.56 (cal/cm$^3$)$^{1/2}$, water tends to penetrate into the separation functional

layer 1 easily. The SP value of B is preferably 9.0 $(cal/cm^3)^{1/2}$ or more, more preferably 11.0 $(cal/cm^3)^{1/2}$ or more, still more preferably 12.0 $(cal/cm^3)^{1/2}$ or more, and particularly preferably 14.0 $(cal/cm^3)^{1/2}$ or more. The upper limit of the SP value of B is not particularly limited, and it may be 30.0 $(cal/cm^3)^{1/2}$, for example. Preferable examples of the SP value of B include 14.0 $(cal/cm^3)^{1/2}$ and 14.51 $(cal/cm^3)^{1/2}$.

B includes, for example, at least one selected from the group consisting of an oxygen atom, a nitrogen atom, a sulfur atom, and a silicon atom. Preferably, B includes at least one selected from the group consisting of an oxygen atom and a nitrogen atom. Particularly preferably, B includes an oxygen atom. B includes, for example, at least one functional group selected from the group consisting of an ether group, an ester group, a ketone group, a hydroxyl group, an amide group, a thioether group, and a sulfonyl group. Preferably, B includes an ether group.

B may include another group, such as a hydrocarbon group, besides the above-mentioned functional groups. As the hydrocarbon group, the hydrocarbon groups stated above for A can be mentioned. B may be identical to or different from A.

In the formula (1), the number of atoms constituting a bonding chain (a shortest bonding chain), among bonding chains that bond $Ar^1$ and $Ar^2$ linked to each other by B, that is composed of a least number of atoms is 1 or more, for example. The upper limit of the number of atoms constituting the shortest bonding chain is not particularly limited, and it is 12, for example. Preferably, the number of atoms constituting the shortest bonding chain is 1.

B may be one of the linking groups 5 to 26 shown in the above-mentioned Tables 1 and 2. B is preferably the linking group 9, the linking group 16, or the linking group 21, and particularly preferably the linking group 21.

In the formula (1), $R^1$ to $R^6$ each are independently a hydrogen atom, a halogen atom, a hydroxyl group, a sulfonic group, an alkoxy group having 1 to 30 carbon atoms, or a hydrocarbon group having 1 to 30 carbon atoms. Preferably, $R^1$ to $R^6$ each are a hydrogen atom. The alkoxy group or the hydrocarbon group as $R^1$ to $R^6$ may be either linear or branched. The number of carbon atoms that the alkoxy group or the hydrocarbon group has is preferably 1 to 20, more preferably 1 to 10, and particularly preferably 1 to 5. Examples of the alkoxy group include a methoxy group, an ethoxy group, and a propoxy group. Examples of the hydrocarbon group include a methyl group, an ethyl group, and a propyl group. At least one hydrogen atom included in the alkoxy group or the hydrocarbon group may be substituted by a halogen atom.

$R^2$ and $R^3$ as well as $R^5$ and $R^6$ may be bonded to each other to form a ring structure. The ring structure is a benzene ring, for example.

In the formula (1), $Ar^1$ and $Ar^2$ each are a divalent aromatic group. The divalent aromatic group includes an aromatic ring. In the formula (1), it is preferable that a nitrogen atom in a phthalimide structure be bonded directly to the aromatic ring included in $Ar^1$, or the aromatic ring included in $Ar^2$. In the formula (1), B may be bonded directly to both of the aromatic ring included in $Ar^1$ and the aromatic ring included in $Ar^2$.

In $Ar^1$ and $Ar^2$, it is preferable that the aromatic ring be composed of a carbon atom. It should be noted that the aromatic ring may be a heteroaromatic ring including a hetero atom such as an oxygen atom, a nitrogen atom, and a sulfur atom. The aromatic ring may be polycyclic, but preferably it is monocyclic. The number of carbon atoms that the aromatic ring has is not particularly limited, and it may be 4 to 14, for example, and it may be 6 to 10. Examples of the aromatic ring include a benzene ring, a naphthalene ring, an anthracene ring, a phenanthrene ring, a furan ring, a pyrrole ring, a pyridine ring, and a thiophene ring.

In $Ar^1$ and $Ar^2$, the aromatic ring may or may not have a substituent. Examples of the substituent of the aromatic ring include a halogen atom, a hydroxyl group, a sulfonic group, an alkoxy group having 1 to 30 carbon atoms, and a hydrocarbon group having 1 to 30 carbon atoms. As the alkoxy group and the hydrocarbon group, the alkoxy groups and the hydrocarbon groups stated above for $R^1$ to $R^6$ can be mentioned. In the case where the aromatic ring has a plurality of substituents, the substituents may be identical to or different from each other.

Preferably, $Ar^1$ and $Ar^2$ each are a phenylene group that may have a substituent, or a naphthalenediyl group that may have a substituent. $Ar^1$ and $Ar^2$ each may be represented by formula (2) below when $Ar^1$ and $Ar^2$ each are a phenylene group that may have a substituent.

[Chemical Formula 4]

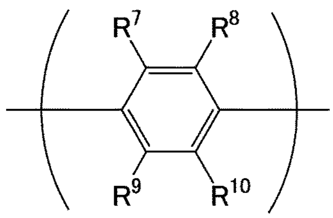

(2)

In the formula (2), $R^7$ to $R^{10}$ each are independently a hydrogen atom, a halogen atom, a hydroxyl group, a

sulfonic group, an alkoxy group having 1 to 30 carbon atoms, or a hydrocarbon group having 1 to 30 carbon atoms. As the alkoxy group and the hydrocarbon group, the alkoxy groups and the hydrocarbon groups stated above for $R^1$ to $R^6$ can be mentioned. Preferably, $R^7$ to $R^{10}$ each are a hydrogen atom. The formula (2) represents a p-phenylene structure. Polyimide having the p-phenylene structure is less bulky three-dimensionally than polyimide having an o-phenylene structure or an m-phenylene structure, and is suitable for enhancing the separation performance of the separation membrane.

The naphthalenediyl group, as $Ar^1$ and $Ar^2$, that may have a substituent has a naphthalene-2,6-diyl structure, a naphthalene-1,4-diyl structure, a naphthalene-1,5-diyl structure, or a naphthalene-1,8-diyl structure, for example. The naphthalenediyl group that may have a substituent is a naphthalene-2,6-diyl group, for example.

$Ar^1$ and $Ar^2$ may be identical to or different from each other. For example, there may be a case in which $Ar^1$ is a naphthalene-2,6-diyl group while $Ar^2$ is a p-phenylene group.

In the polyimide (P), the structural unit represented by the formula (1) is preferably a structural unit represented by formula (3) below.

[Chemical Formula 5]

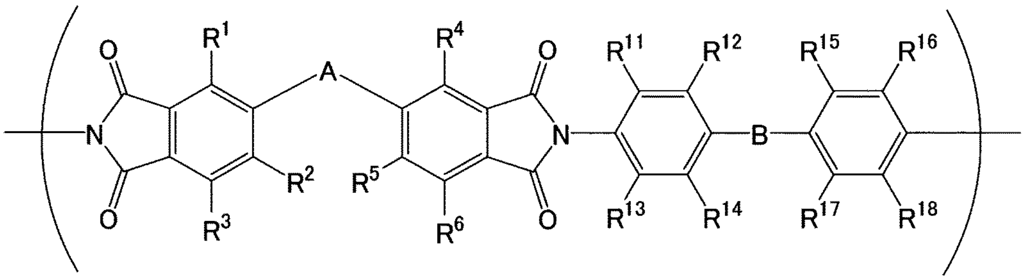

(3)

In the formula (3), A, B, and $R^1$ to $R^6$ are identical to those mentioned above for the formula (1). $R^{11}$ to $R^{18}$ each are independently a hydrogen atom, a halogen atom, a hydroxyl group, a sulfonic group, an alkoxy group having 1 to 30 carbon atoms, and a hydrocarbon group having 1 to 30 carbon atoms. As the alkoxy group and the hydrocarbon group, the alkoxy groups and the hydrocarbon groups stated above for $R^1$ to $R^6$ can be mentioned. $R^{11}$ to $R^{18}$ each are preferably a hydrogen atom.

A content of the structural unit represented by the formula (1) in the polyimide (P) is 50 mol% or more, for example, and it is preferably 60 mol% or more, more preferably 70 mol% or more, still more preferably 80 mol% or more, and particularly preferably 90 mol% or more. The content of the structural unit represented by the formula (1) may be 100 mol%.

The structural unit represented by the formula (1) can be obtained by a reaction between tetracarboxylic dianhydride (C) represented by formula (4) below and a diamine compound (D) represented by formula (5) below. In the formula (4), A as well as $R^1$ to $R^6$ are identical to those in the formula (1). In the formula (5), B, $Ar^1$, and $Ar^2$ are identical to those in the formula (1).

[Chemical Formula 6]

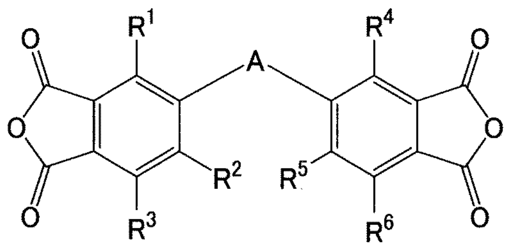

(4)

[Chemical Formula 7]

$H_2N\text{-}Ar^1\text{-}B\text{-}Ar^2\text{-}NH_2$ (5)

The polyimide (P) may include a structural unit derived from an other tetracarboxylic dianhydride that is different from the tetracarboxylic dianhydride (C). The other tetracarboxylic dianhydride is not particularly limited, and a known tetracarboxylic dianhydride can be used. Examples of the other tetracarboxylic dianhydride include pyromellitic dianhydride, and 4,4'-(hexafluoroisopropylidene)diphthalic anhydride.

In the polyimide (P), a ratio P1 of a structural unit(s) derived from the tetracarboxylic dianhydride (C) with respect to structural units derived from all the tetracarboxylic dianhydrides is 50 mol% or more, for example, and it is preferably 70 mol% or more, and more preferably 90 mol% or more. The ratio P1 may be 100 mol%.

The polyimide (P) may include a structural unit derived from an other diamine compound that is different from the diamine compound (D). The other diamine compound is not particularly limited and a known diamine compound can be used. Examples of the other diamine compound include phenylenediamine, diaminobenzoic acid, diaminobiphenyl, and diaminodiphenylmethane. For example, the polyimide (P) may include a structural unit derived from diaminobenzoic acid (such as 3,5-diaminobenzoic acid). The polyimide (P) including the structural unit derived from diaminobenzoic acid is suitable for increasing a flux of the water permeating through the separation membrane 10.

In the polyimide (P), a ratio P2 of a structural unit(s) derived from the diamine compound (D) with respect to structural units derived from all the diamine compounds is 50 mol% or more, for example, and it is preferably 70 mol% or more, and more preferably 90 mol% or more. The ratio P2 may be 100 mol%.

The polyimide (P) can be produced by the following method, for example. First, the diamine compound (D) is dissolved in a solvent to obtain a solution. Examples of the solvent include a polar organic solvent such as N-methyl-2-pyrrolidone (NMP) and 1,3-dioxolane. Next, the tetracarboxylic dianhydride (C) is added gradually to the obtained solution. This makes the tetracarboxylic dianhydride (C) and the diamine compound (D) react with each other to form polyamide acid. The addition of the tetracarboxylic dianhydride (C) is carried out under the conditions, for example, that the solution is being stirred for 3 to 20 hours at a temperature equal to or lower than a room temperature (25°C).

Subsequently, the polyamide acid is imidized to obtain the polyimide (P). Examples of the imidization method include a chemical imidization method and a thermal imidization method. The chemical imidization method is a method for imidizing polyamide acid by using a dehydration condensation agent. The chemical imidization method may be carried out under a room temperature condition or under a heating condition. Examples of the dehydration condensation agent include acetic anhydride, pyridine, and triethylamine. The thermal imidization method is a method for imidizing polyamide acid by a heat treatment. The heat treatment is carried out at a temperature of 180°C or higher, for example.

A content of the polyimide (P) in the matrix 4 is 50 wt% or more, for example, and it is preferably 60 wt% or more, more preferably 70 wt% or more, still more preferably 80 wt% or more, and particularly preferably 90 wt% or more. The matrix 4 is composed substantially of the polyimide (P).

A content of the matrix 4 in the separation functional layer 1 is 70 wt% or more, for example. The upper limit of the content of the matrix 4 is not particularly limited, and it may be 99 wt% or 95 wt%.

The separation functional layer 1 has a thickness that is not particularly limited. It is 50 μm or less, for example, and it is preferably 30 μm or less, and more preferably 20 μm or less. The thickness of the separation functional layer 1 may be 1 μm or more, 5 μm or more, or 10 μm or more. In the separation membrane 10 of the present embodiment, even when the thickness of the separation functional layer 1 is relatively large, the flux of the water permeating through the separation membrane 10 tends to be sufficiently high.

The porous support member 2 is not particularly limited as long as it can support the separation functional layer 1. Examples of the porous support member 2 include: a nonwoven fabric; porous polytetrafluoroethylene; aromatic polyamide fiber; a porous metal; a sintered metal; porous ceramic; porous polyester; porous nylon; activated carbon fiber; latex; silicone; silicone rubber; a permeable (porous) polymer including at least one selected from the group consisting of polyvinyl fluoride, polyvinylidene fluoride, polyurethane, polypropylene, polyethylene, polycarbonate, polysulfone, polyether ether ketone, polyacrylonitrile, polyimide, and polyphenylene oxide; a metallic foam having an open cell or a closed cell; a polymer foam having an open cell or a closed cell; silica; porous glass; and a mesh screen. The porous support member 2 may be a combination of two or more of these materials.

The porous support member 2 has an average pore diameter of 0.01 to 0.4 μm, for example. The porous support member 2 has a thickness that is not particularly limited. It is 10 μm or more, for example, and it is preferably 20 μm or more, and more preferably 50 μm or more. The thickness of the porous support member 2 is 300 μm or less, for example, and it is preferably 200 μm or less, and more preferably 75 μm or less.

The separation membrane 10 can be produced by forming the separation functional layer 1 on the porous support member 2. The separation functional layer 1 can be produced by the following method, for example. First, a coating liquid containing the filler 3 and the material of the matrix 4 is prepared. As a solvent of the coating liquid, an organic solvent, such as 1,3-dioxolane, can be used. The coating liquid may be subject to ultrasonication in order to enhance the dispersibility of the filler 3 in the coating liquid. Next, the coating liquid is applied onto the porous support member 2 to obtain a coating. The coating is dried to form the separation functional layer 1. Thereby, the separation membrane 10 can be produced.

In the case where the matrix 4 contains polyimide, the material of the matrix 4 contained in the coating liquid

may be polyamide acid. In this case, the separation functional layer 1 can be formed by imidizing the polyamide acid after applying the coating liquid onto the porous support member 2.

The separation membrane 10 of the present embodiment is used for, for example, separating water from a liquid mixture containing an alcohol and water. In this use, the separation membrane 10 is suitable for increasing a flux of the water permeating through the separation membrane 10. However, the use of the separation membrane 10 is not limited to the above-mentioned use of separating water from a liquid mixture. In the present description, the "flux of water" means a weight of the water permeating through the separation membrane 10 for 1 hour when the separation membrane 10 has an area of 1 $m^2$. For example, when, in a state in which a liquid mixture composed of ethanol and water is in contact with one surface of the separation membrane 10, a space adjacent to an other surface of the separation membrane 10 is decompressed, a flux F of the water permeating through the separation membrane 10 is 0.12 $kg/m^2/hr$ or more, for example, and it is preferably 0.15 $kg/m^2/hr$ or more, more preferably 0.20 $kg/m^2/hr$ or more, and still more preferably 0.25 $kg/m^2/hr$ or more. According to the separation membrane 10 of the present embodiment, the flux F of the water can be adjusted to 0.12 $kg/m^2/hr$ or more in some cases even when the thickness of the separation functional layer 1 is 10 μm or more.

Specifically, the flux F of the water can be measured by the following method.

First, in a state in which a liquid mixture composed of ethanol and water is in contact with one surface (a principal surface 11, on a side of the separation functional layer, of the separation membrane 10, for example) of the separation membrane 10, a space adjacent to an other surface (a principal surface 12, on a side of the porous support member, of the separation membrane 10, for example) of the separation membrane 10 is decompressed. Thereby, a permeation fluid that has permeated through the separation membrane 10 can be obtained. In the above-mentioned procedure, a concentration of the ethanol in the liquid mixture is 50 vol% (44 wt%) when measured with a temperature of the liquid mixture at 20°C. The liquid mixture in contact with the separation membrane 10 has a temperature of 60°C. The space adjacent to the other surface of the separation membrane 10 is decompressed in such a manner that a pressure in the space is lower than an atmospheric pressure in a measurement environment by 100 kPa. Next, a weight of the permeation fluid and a weight ratio of the water in the permeation fluid are measured. The flux F of the water can be determined based on the obtained results.

It is preferable that the flux F of the water be evaluated taking the thickness of the separation functional layer 1 into consideration. For example, a product of the flux F of the water and a thickness T (μm) of the separation functional layer 1 is 1.5 $μm•kg/m^2/hr$ or more, for example, and it is preferably 2.0 $μm•kg/m^2/hr$ or more, more preferably 2.5 $μm•kg/m^2/hr$ or more, and still more preferably 3.0 $μm•kg/m^2/hr$ or more. The upper limit of the product of the flux F of the water and the thickness T of the separation functional layer 1 is not particularly limited, and it is 20$μm•kg/m^2/hr$, for example.

Under the above-mentioned measurement conditions of the flux F of the water, a separation factor α that the separation membrane 10 has for water with respect to ethanol is 20 or more, for example, and it is preferably 30 or more, more preferably 40 or more, still more preferably 50 or more, particularly preferably 60 or more, and especially preferably 80 or more. The upper limit of the separation factor α Is not particularly limited, and it is 500, for example. The separation membrane 10 having the separation factor α that is 20 or more is sufficiently suitable to be used for separating water from a liquid mixture containing an alcohol and water.

The separation factor α can be calculated by the following formula. It should be noted that in the following formula, $X_A$ and $X_B$ are respectively a volume ratio of the water and a volume ratio of the alcohol in the liquid mixture. $Y_A$ and $Y_B$ are respectively the volume ratio of the water and the volume ratio of the alcohol in the permeation fluid that has permeated through the separation membrane 10.

$$\text{Separation factor } \alpha = (Y_A/Y_B)/(X_A/X_B)$$

As described above, the separation membrane 10 of the present embodiment is suitable for, in the case of separating water from a liquid mixture containing an alcohol and water, increasing the flux of the water permeating through the separation membrane 10. This characteristic derives from the metal organic framework S1 included in the separation membrane 10. The ratio R1 and ratio R2 of the metal organic framework each are an index as to adsorptivity of water by the metal organic framework. However, the studies by the present inventors have found that the ratio R1 and the ratio R2 each are also useful as an index as to the flux of the water permeating through the separation membrane.

The metal organic framework S1 mentioned above can also be used for, in an embodiment other than the separation membrane 10, separating water from a liquid mixture containing an alcohol and water. For example, when the filler 3 mentioned above is added directly to a liquid mixture containing an alcohol and water, the filler 3 adsorbs preferentially or selectively the water contained in the liquid mixture. Thereby, the water can be separated from the liquid mixture. In this manner, the filler 3 including the metal organic framework S1 can also function as an adsorbent for water. A drying treatment on the filler 3 that has adsorbed water allows the filler 3 to be used repeatedly. The present invention

provides, in another aspect, the metal organic framework S1 used for separating water from a liquid mixture containing an alcohol and water, wherein at least one of the requirements (i) and (ii) mentioned above holds.

(Embodiment 2)

Next, one embodiment of the separation membrane provided from the second aspect will be described. The separation membrane of the present embodiment has the same structure as that of the separation membrane of Embodiment 1, except that it includes a metal organic framework S2 instead of the metal organic framework S1. Therefore, the separation membrane of the present embodiment will also, in some cases, be described with reference to FIG. 1 in the same manner as the separation membrane of Embodiment 1.

As shown in FIG. 1, the separation membrane 10 of the present embodiment is provided with the separation functional layer 1 having the filler 3 and the matrix 4. The filler 3 includes the metal organic framework S2.

In the present embodiment, a crystal structure of the metal organic framework S2 has a volume change ratio W1, calculated by a molecular simulation, of 0% or less with respect to ethanol. The volume change ratio W1 is preferably -3.0% or less, more preferably -5.5% or less, still more preferably -8.0% or less, and particularly preferably -10.0% or less. The lower limit of the volume change ratio W1 is not particularly limited, and it is -50.0%, for example. The volume change ratio W1 tends to be affected by a factor such as a chemical interaction between the metal organic framework S2 and an ethanol molecule(s). That the volume change ratio W1 is a negative value means that the crystal structure of the metal organic framework S2 is in contact with ethanol and shrinks by allowing the ethanol molecule(s) to be sorbed to an inside of the crystal structure. The separation membrane 10 including the metal organic framework S2 having the volume change ratio W1 that is 0% or less tends to exhibit high separation performance when separating water from a liquid mixture containing an alcohol and water.

The molecular simulation for calculating the volume change ratio W1 can be carried out using, for example, Materials Studio available from Dassault Systemes, Gaussian09 available from Gaussian, Inc., and a known software such as LAMMPS distributed by Sandia National Laboratories of the US Department of Energy.

Hereinafter, an example of a method for determining the volume change ratio W1 will be described. First, a crystal structure model of the metal organic framework S2 is created by the method mentioned above in Embodiment 1 to calculate a potential energy of the model. The obtained potential energy can be assumed as a potential energy $E_{MOF2}$ of the metal organic framework S2 in an isolated state. Next, a molecular model of ethanol is created by the method mentioned above in Embodiment 1, and a potential energy of the molecular model is calculated. A potential energy of two or more ethanol molecules is calculated by multiplying the potential energy of ethanol per molecule by the number of the ethanol molecules. The obtained potential energy can be assumed as the potential energy $E_{EtOH}$ of the ethanol molecule(s) in an isolated state.

Next, a complex model in which the molecular model of ethanol is inserted inside the crystal structure model of the metal organic framework S2 is created by the method mentioned above in Embodiment 1. As stated herein, a plurality of the complex models in which the molecular model(s) is/are inserted in different numbers, respectively, are created. Next, a potential energy of each of the complex models is calculated by the above-mentioned method. The obtained potential energy of each of the complex models can be assumed as a potential energy $E_{MOF2 + ETOH}$ of a composite composed of the metal organic framework S2 and the ethanol molecule(s).

Next, a value ΔE3 is calculated by subtracting the total value of the potential energy $E_{MOF2}$ of the metal organic framework S2 and the potential energy $E_{EtOH}$ of the ethanol molecule(s) from the potential energy $E_{MOF2 + EtOH}$ of the composite. Specifically, the value ΔE3 can be calculated by formula (III) below. $E_{EtOH}$ in the formula (III) means a potential energy of the same number of ethanol molecules in an isolated state as the number of the ethanol molecules included in the composite. The value ΔE3 represents a potential energy change caused by the insertion of the ethanol molecule(s).

$$\Delta E3 = E_{MOF2 + EtOH} - (E_{MOF2} + E_{EtOH}) \qquad (III)$$

Next, a graph showing a relationship between a number N of the molecular models of ethanol (a number N of the ethanol molecules) inserted inside the metal organic framework S2 and the value ΔE3 is created based on the obtained result. Next, a range in which the value ΔE3 is a negative value is determined from this graph. That the value ΔE3 is a negative value means the potential energy $E_{MOF2 + ETOH}$ of the composite is less than the total value of the potential energy $E_{MOF2}$ of the metal organic framework S2 and the potential energy $E_{EtOH}$ of the ethanol molecule(s). Next, a number N3 of the molecular models of ethanol when the value ΔE3 is minimum (smallest), that is, when a value |ΔE3| is maximum, in the range in which the value ΔE3 is a negative value is determined. It should be noted that a metal organic framework with the value ΔE3 that fails to be a negative value (with the value ΔE3 that is always a positive value) is not suitable for the separation membrane of the present embodiment.

Next, a diffusion simulation is carried out using a complex model in which the number N3 of the molecular

models of ethanol is/are inserted inside the crystal structure model of the metal organic framework S2. The diffusion simulation can be carried out by making a molecular dynamics calculation on the complex model using an NPT ensemble (an isothermal-isobaric ensemble). For the molecular dynamics calculation, the universal force field (UFF) can be used as a molecular force field. Specifically, a mean square displacement data of ethanol is acquired by making a calculation at a time scale of 5 to 10 ns and at 1 fs intervals under conditions that a pressure is 1 atm and a temperature is 298K. A diffusion coefficient $D_{EtOH}$ of the ethanol in the complex model is represented by formula (IV) below. In the formula (IV), $\langle|r(t) - r(0)|^2\rangle$ means a mean square displacement of the ethanol molecule(s) (particle(s)) in t hour(s) from the start of the calculation.
[Formula 1]

$$D_{EtOH} = \frac{1}{6}\langle|r(t) - r(0)|^2\rangle \qquad (IV)$$

Next, by the molecular dynamics calculation, a lattice volume V1 of the metal organic framework S2 when the molecular model(s) of ethanol is/are diffused inside the metal organic framework S2 and sorbed to the inside of the metal organic framework S2 and the sorption has reached a state of equilibrium is determined. The lattice volume V1 is a value in a state in which no time-dependent change (no tendency of increase or decrease) in the lattice volume of the metal organic framework S2 is observed when the molecular dynamics calculation is made in the range of 5 to 10 ns. Specifically, the lattice volume V1 can be determined by the following method. First, the molecular dynamics calculation is made to create a graph that shows a relationship between lapsed time and the lattice volume of the metal organic framework S2. From the graph, a range in which no time-dependent change in the lattice volume is substantially observed is determined. Data at at least 50 points within this range are selected, and an average of these lattice volume data can be determined as the lattice volume V1. The volume change ratio W1 can be calculated by formula (V) below based on the lattice volume V1 and a lattice volume V0 of the metal organic framework S2 before the molecular dynamics calculation is made.

$$W1\ (\%) = 100 \times (V1 - V0)/V0 \qquad (V)$$

In the present embodiment, a volume change ratio W2, calculated by the molecular simulation, of the crystal structure of the metal organic framework S2 with respect to water is not particularly limited, and it is 0% or more, for example, and it is preferably 3.0% or more. It may be 5.0% or more, or 10.0% or more. The upper limit of the volume change ratio W2 is not particularly limited, and it is 50.0%, for example, and it may be 30.0%. The volume change ratio W2 tends to be affected by a factor such as a chemical interaction between the metal organic framework S2 and the water molecule(s). That the volume change ratio W2 is a positive value means that the crystal structure of the metal organic framework S2 is in contact with water and expands by allowing the water molecule(s) to be sorbed to the inside of the crystal structure. The separation membrane 10 including the metal organic framework S2 having the volume change ratio W2 that is 0% or more is suitable for, in the case of separating water from a liquid mixture containing an alcohol and water, increasing the flux of the water permeating through the separation membrane 10.

The volume change ratio W2 can be calculated by the same method as that used for the volume change ratio W1. For example, the volume change ratio W2 can be determined by the following method. First, the crystal structure model of the metal organic framework S2 is created by the method mentioned above, and the potential energy $E_{MOF2}$ of the metal organic framework S2 in an isolated state is determined. Next, a molecular model of water is created by the method mentioned above in Embodiment 1, and a potential energy of the molecular model is calculated. A potential energy of two or more water molecules is calculated by multiplying the potential energy of water per molecule by the number of the water molecules. The obtained potential energy can be assumed as the potential energy $E_{H2O}$ of the water molecule(s) in an isolated state.

Next, a complex model in which the molecular model of water is inserted inside the crystal structure model of the metal organic framework S2 is created by the method mentioned above in Embodiment 1. As stated herein, a plurality of the complex models in which the molecular model(s) is/are inserted in different numbers, respectively, are created. Next, a potential energy of each of the complex models is calculated by the above-mentioned method. The obtained potential energy of each of the complex models can be assumed as the potential energy $E_{MOF2 + H2O}$ of the composite composed of the metal organic framework S2 and the water molecule(s).

Next, a value $\Delta E4$ is calculated by subtracting the total value of the potential energy $E_{MOF2}$ of the metal organic framework S2 and the potential energy $E_{H2O}$ of the water molecule(s) from the potential energy $E_{MOF2 + H2O}$ of the composite. Specifically, the value $\Delta E4$ can be calculated by formula (VI) below. $E_{H2O}$ in the formula (VI) means a potential energy of the same number of water molecules in an isolated state as the number of the water molecules included in

the composite. The value ΔE4 represents a potential energy change caused by the insertion of the water molecule(s).

$$\Delta E4 = E_{MOF2 + H2O} - (E_{MOF2} + E_{H2O}) \qquad (VI)$$

Next, a graph showing a relationship between a number N of the molecular models of water (a number N of the water molecules) inserted inside the metal organic framework S2 and the value ΔE4 is created based on the obtained result. Next, a range in which the value ΔE4 is a negative value is determined from this graph. That the value ΔE4 is a negative value means the potential energy $E_{MOF2} + {}_{H2O}$ of the composite is less than the total value of the potential energy $E_{MOF2}$ of the metal organic framework S2 and the potential energy $E_{H2O}$ of the water molecule(s). Next, a number N4 of the molecular models of water when the value ΔE4 is minimum (smallest), that is, when a value |ΔE4| is maximum, in the range in which the value ΔE4 is a negative value is determined.

Next, a diffusion simulation is carried out using a complex model in which the number N4 of the molecular models of ethanol is/are inserted inside the crystal structure model of the metal organic framework S2. The diffusion simulation can be performed by the method mentioned above. A diffusion coefficient $D_{H2O}$ of the water in the complex model is represented by formula (VII) below. In the formula (VII), $\langle |r(t) - r(0)|^2 \rangle$ means a mean square displacement of the water molecule(s) (particle(s)) in t hour(s) from the start of the calculation.

[Formula 2]

$$D_{H2O} = \frac{1}{6} \langle |r(t) - r(0)|^2 \rangle \qquad (VII)$$

Next, by the molecular dynamics calculation, a lattice volume V2 of the metal organic framework S2 when the molecular model(s) of water is/are diffused inside the metal organic framework S2 and sorbed to the inside of the metal organic framework S2 and the sorption has reached a state of equilibrium is determined. The lattice volume V2 is a value in a state in which no time-dependent change (no tendency of increase or decrease) in the lattice volume of the metal organic framework S2 is observed when the molecular dynamics calculation is made in the range of 5 to 10 ns. Specifically, the lattice volume V2 can be determined by the method mentioned above for the lattice volume V1. The volume change ratio W2 can be calculated by formula (VIII) below based on the lattice volume V2 and the lattice volume V0 of the metal organic framework S2 before the molecular dynamics calculation is made.

$$W2\ (\%) = 100 \times (V2 - V0)/V0 \qquad (VIII)$$

The metal organic framework S2 has a void fraction of 35% or more, for example, and it is preferably 40% or more, more preferably 50% or more, and still more preferably 60% or more. The upper limit of the void fraction of the metal organic framework S2 is not particularly limited, and it is 75%, for example. The metal organic framework S2 has a largest cavity diameter of 0.5 nm or more, for example, and it is preferably 0.7 nm or more, and more preferably 1.0 nm or more. The upper limit of the largest cavity diameter of the metal organic framework S2 is not particularly limited, and it is 2.0 nm, for example. The void fraction and the largest cavity diameter of the metal organic framework S2 can be determined by the method mentioned above for the metal organic framework S1.

The metal organic framework S2 includes a metal ion and an organic ligand. Examples of the metal ion included in the metal organic framework S2 include a Pb ion and a Cu ion. The organic ligand may have a polar group. Examples of the polar group include a carboxyl group, a hydroxyl group, and an ester group. The larger the number of the polar groups included in the organic ligand is, the smaller the volume change ratio W1 of the metal organic framework S2 tends to be. Examples of the organic ligand included in the metal organic framework S2 include: polysaccharides such as cyclodextrin; a lactone compound such as ascorbic acid; and a carboxylic acid compound such as benzene-1,3,5-tricarboxylic acid.

The metal organic framework S2 may be one of those mentioned as the examples of the metal organic framework S1. The metal organic framework S2 includes, for example, at least one selected from the group consisting of TEVZIF, JEDKIM, and HKUST-1(copper benzen-1,3,5-tricarboxylate), and preferably it includes at least one selected from the group consisting of TEVZIF and JEDKIM.

The filler 3 includes the metal organic framework S2 as a main component, for example. A content of the metal organic framework S2 in the filler 3 is 50 wt% or more, for example, and it is preferably 80 wt% or more, and more preferably 95% or more. The filler 3 is substantially composed of the metal organic framework S2, for example. The filler 3 may include a component other than the metal organic framework S2.

As in Embodiment 1, the separation membrane 10 of the present embodiment is used for, for example, separating

water from a liquid mixture containing an alcohol and water. In this use, the separation membrane 10 of the present embodiment tends to exhibit high separation performance. However, the use of the separation membrane 10 is not limited to the use of separating water from the above-mentioned liquid mixture. For example, the separation factor $\alpha$ of the separation membrane 10 with respect to ethanol under the measurement conditions mentioned above in Embodiment 1 is 40 or more, for example, and it is preferably 60 or more, more preferably 80 or more, and still more preferably 100 or more. The upper limit of the separation factor $\alpha$ is not particularly limited, and it is 500, for example.

Furthermore, the flux F of the water permeating through the separation membrane 10 under the measurement conditions mentioned above in Embodiment 1 is 0.09 $kg/m^2/hr$ or more, for example, and it is preferably 0.10 $kg/m^2/hr$, and more preferably 0.15 $kg/m^2/hr$ or more. The product of the flux F of the water and the thickness T ($\mu m$) of the separation functional layer 1 is 1.0 $\mu m \cdot kg/m^2/hr$ or more, for example, and it is preferably 1.5 $\mu m \cdot kg/m^2/hr$ or more, and more preferably 2.0 $\mu m \cdot kg/m^2/hr$ or more. The upper limit of the product of the flux F of the water and the thickness T of the separation functional layer 1 is not particularly limited, and it is 20 $\mu m \cdot kg/m^2/hr$, for example.

As described above, the separation membrane 10 of the present embodiment tends to exhibit high separation performance in the case of separating water from a liquid mixture containing an alcohol and water. This characteristic derives from the metal organic framework S2 included in the separation membrane 10. The volume change ratio W1 of the metal organic framework is an index as to a behavior of the metal organic framework with respect to ethanol. However, the studies by the present inventors have found that the volume change ratio W1 is also useful as an index as to the separation performance of the separation membrane in the case of separating water from a liquid mixture containing an alcohol and water.

The metal organic framework S2 mentioned above can also be used for, in an embodiment other than the separation membrane 10, separating water from a liquid mixture containing an alcohol and water. For example, when the filler 3 including the metal organic framework S2 is added directly to a liquid mixture containing an alcohol and water, the filler 3 adsorbs preferentially or selectively the water contained in the liquid mixture. Thereby, the water can be separated from the liquid mixture. In this manner, the filler 3 including the metal organic framework S2 can also function as an adsorbent for water. A drying treatment on the filler 3 that has adsorbed water allows the filler 3 to be used repeatedly. The present invention provides, in another aspect, the metal organic framework S2 used for separating water from a liquid mixture containing an alcohol and water, wherein the crystal structure of the metal organic framework S2 has a volume change ratio, calculated by the molecular simulation, of 0% or less with respect to ethanol.

(Embodiment of membrane separation device)

As shown in FIG. 3, a membrane separation device 100 of the present embodiment is provided with the separation membrane 10 and a tank 20. The separation membrane 10 may be the one mentioned above in Embodiment 1, or the one mentioned above in Embodiment 2. The tank 20 is provided with a first room 21 and a second room 22. The separation membrane 10 is disposed in the tank 20. In the tank 20, the separation membrane 10 separates the first room 21 from the second room 22. The tank 20 has a pair of wall surfaces, and the separation membrane 10 extends from one of them to the other.

The first room 21 has an inlet 21a and an outlet 21b. The second room 22 has an outlet 22a. The inlet 21a, the outlet 21b, and the outlet 22a each are an opening formed in the wall surfaces of the tank 20, for example.

Membrane separation using the membrane separation device 100 is carried out by the following method, for example. First, a liquid mixture 30 containing an alcohol and water is supplied into the first room 21 via the inlet 21a. This makes it possible to bring the liquid mixture 30 into contact with one surface of the separation membrane 10. The alcohol contained in the liquid mixture 30 is, for example, a lower alcohol that exhibits azeotropy with water. The alcohol is preferably ethanol, but it may be isopropyl alcohol (IPA). A concentration of the alcohol in the liquid mixture 30 is 10 wt% or more, for example, and it is preferably 20 wt% or more. The separation membrane 10 is particularly suitable for separating the water from the liquid mixture 30 containing the alcohol at a moderate concentration (20 wt% to 80 wt%, particularly 30 wt% to 70 wt%). It should be noted that the concentration of the alcohol in the liquid mixture 30 may be 80 wt% or more. The liquid mixture 30 may be composed substantially of the alcohol and water. A temperature of the liquid mixture 30 may be higher than a boiling point of the alcohol to be used. Preferably, the temperature is lower than the boiling point of the alcohol. The temperature of the liquid mixture 30 is 25°C or higher, for example, and it is preferably 40°C or higher, and more preferably 60°C or higher. The temperature of the liquid mixture 30 may be 75°C or lower.

Next, in a state in which the liquid mixture 30 is in contact with one surface of the separation membrane 10, a space adjacent to an other surface of the separation membrane 10 is decompressed. Specifically, an inside of the second room 22 is decompressed via the outlet 22a. The membrane separation device 100 may be further provided with a pump (not shown) for decompressing the inside of the second room 22. The second room 22 is decompressed in such a manner that a space in the second room 22 has a pressure lower than an atmospheric pressure in a measurement environment by 10 kPa or more, for example, and preferably by 50 kPa or more, and more preferably by 100 kPa or more.

Decompressing the inside of the second room 22 makes it possible to obtain, on a side of the other surface of

the separation membrane 10, a permeation fluid 35 having a content of the water higher than a content of the water in the liquid mixture 30. That is, the permeation fluid 35 is supplied into the second room 22. The permeation fluid 35 contains the water as a main component, for example. The permeation fluid 35 may contain a small amount of the alcohol besides the water. The permeation fluid 35 may be a gas or a liquid. The permeation fluid 35 is discharged to an outside of the tank 20 via the outlet 22a.

The concentration of the alcohol in the liquid mixture 30 increases gradually from the inlet 21a toward the outlet 21b of the first room 21. The liquid mixture 30 (a concentrated fluid 36) processed in the first room 21 is discharged to the outside of the tank 20 via the outlet 21b.

The membrane separation device 100 of the present embodiment is used preferably for a pervaporation method. The membrane separation device 100 may be used for other membrane separation methods such as a vapor permeation method. That is, a mixture gas containing a gaseous alcohol and gaseous water may be used instead of the liquid mixture 30 in the membrane separation method mentioned above. The membrane separation device 100 of the present embodiment is suitable for a flowtype (continuous-type) membrane separation method. The membrane separation device 100 of the present embodiment may be used for a batch-type membrane separation method.

(Modification of membrane separation device)

As shown in FIG. 4, a membrane separation device 110 of the present embodiment is provided with a central tube 41 and a laminate 42. The laminate 42 includes the separation membrane 10. The membrane separation device 110 is a spiral membrane element.

The central tube 41 has a cylindrical shape. The central tube 41 has a surface with a plurality of pores formed therein to allow the permeation fluid 35 to flow into the central tube 41. Examples of a material of the central tube 41 include: a resin such as an acrylonitrile-butadiene-styrene copolymer (an ABS resin), a polyphenylene ether resin (a PPE resin), and a polysulfone resin (a PSF resin); and a metal such as stainless steel and titanium. The central tube 41 has an inner diameter in a range of 20 to 100 mm, for example.

The laminate 42 further includes a supply-side flow passage material 43 and a permeation-side flow passage material 44 besides the separation membrane 10. The laminate 42 is wound around a circumference of the central tube 41. The membrane separation device 110 may be further provided with an exterior material (not shown).

As the supply-side flow passage material 43 and the permeation-side flow passage material 44, a resin net composed of polyphenylene sulfide (PPS) or an ethylene-chlorotrifluoroethylene copolymer (ECTFE) can be used, for example.

Membrane separation using the membrane separation device 110 is carried out by the following method, for example. First, the liquid mixture 30 is supplied into an end of the wound laminate 42. An inner space of the central tube 41 is decompressed. Thereby, the permeation fluid 35 that has permeated through the separation membrane 10 of the laminate 42 moves into the central tube 41. The permeation fluid 35 is discharged to an outside via the central tube 41. The liquid mixture 30 (the concentrated fluid 36) processed by the membrane separation device 110 is discharged to the outside from an other end of the wound laminate 42. Thereby, the water can be separated from the liquid mixture 30.

EXAMPLES

Hereinafter, the present invention will be described in detail by way of examples and comparative examples. It should be noted that the present invention is not limited to these examples.

(Example 1)

First, as tetracarboxylic dianhydride, bis(1,3-dioxo-1,3-dihydroisobenzofuran-5-carboxylic acid) ethylene (RIKACID TMEG-100, a compound represented by the formula (4) where A was the linking group 18 as well as $R^1$ to $R^6$ each were a hydrogen atom) was prepared. The RIKACID TMEG-100 was subject to a drying treatment under a vacuum atmosphere for 8 hours using a vacuum dryer set at 100°C. Next, as diamine compounds, 4,4'-diaminodiphenyl ether (44'DDE, a compound represented by the formula (5) where B was the linking group 21 as well as $Ar^1$ and $Ar^2$ each were a p-phenylene group), and 3,5-diaminobenzoic acid (DBA) were prepared. These diamine compounds were subject to a drying treatment under a vacuum atmosphere for 7 hours using a vacuum dryer set at a room temperature. In addition, NMP (super-dehydrated) was prepared as a reactional solvent. The NMP was subject to a dehydrating treatment by adding a molecular sieve.

Subsequently, a 500 mL separable flask was equipped with a stirring blade made of Teflon (registered trademark) and a calcium chloride tube filled with a silica gel. Nitrogen gas was introduced into the separable flask. Approximately 87 g of the NMP was added into the separable flask and stirred with the stirring blade. The stirring with the stirring blade was carried out at a revolving speed of 150 rpm using a mechanical stirrer. Next, 10.81 g (54 mmol) of the 44'DDE and

0.91 g (6 mmol) of the DBA were added into the separable flask. Approximately 5 minutes of stirring by the stirring blade allowed most of these diamine compounds to be dissolved in the NMP. Subsequently, 24.61 g (60 mmol) of the RIKACID TMEG-100 was added into the separable flask. This initiated a reaction between the RIKACID TMEG-100 and the diamine compounds. After the RIKACID TMEG-100 was added, it was confirmed, by touching an outer wall of the flask, that the resultant reaction mixture was generating heat. When reagents such as the diamine compounds and the RIKACID TMEG-100 were added into the separable flask, an inside of each of containers in which these reagents had been stored was rinsed with an additional portion of the NMP. Thus, the NMP added into the separable flask was 109.03 g in total. The stirring by the stirring blade was carried out at a room temperature for 22 hours after the RIKACID TMEG-100 had been added. Since a viscosity of the reaction mixture increased during the stirring, the revolving speed for stirring was lowered to 100 rpm. A solution of polyamide acid was obtained by the reaction between the RIKACID TMEG-100 and the diamine compounds. A content of the polyamide acid in the solution was 25 wt%.

Subsequently, the polyamide acid was chemically-imidized by the following method using a half amount of the obtained solution. First, the NMP was added to the solution to adjust the concentration of the polyamide acid in the solution to 10 wt%. The solution of polyamide acid, acetic anhydride, and triethylamine were added in this order into a reaction vessel. In the obtained mixed solution, a molar ratio among the polyamide acid, the acetic anhydride, and the triethylamine was 1:3:0.5. The mixed solution was stirred for 6 hours with a stirring blade at a revolving speed of 100 rpm while being heated using a 60°C oil bath. Thereby, the polyamide acid was chemically-imidized. The heating process was stopped, and then the obtained reaction mixture was diluted so that the polyimide had a concentration of 4 wt%. Subsequently, a reprecipitation process was carried out using ion exchanged water in an amount approximately 3.5 times larger than that of the reaction mixture. The obtained precipitate was filtered out using a Buchner funnel. The precipitate was stir-washed with ion exchanged water and filtered out, and this process was repeated three times in total, followed by being washed with methanol. The precipitate was dried for 15 hours in a hot air circulation dryer set at 60°C, and then dried for 8 hours in a vacuum dryer set at 60°C. Thereby, a desired product (polyimide) was obtained in an amount of 19.2 g (95.9% yield).

Next, a filler (available from PlasmaChem GmbH) composed of MAF-Mg was prepared. This filler was added to 1,3-dioxolane and the resultant mixture was subject to ultrasonication for 30 minutes to prepare a dispersion of the filler. Subsequently, the polyimide mentioned above was added to 1,3-dioxolane to prepare a solution of the polyimide. The solution of the polyimide was added to the dispersion of the filler, and the resultant mixture was subject to ultrasonication (with an ultrasonic homogenizer at a power of 92%) for 5 minutes to prepare a coating liquid. The coating liquid had a solid content concentration of 10 wt%. In the coating liquid, a weight ratio between the polyimide and the MAF-Mg was 9:1.

Next, the coating liquid was applied onto a porous support member to obtain a coating. As the porous support member, a UF membrane (ultrafiltration membrane) RS-50 (a laminate of a PVDF porous layer and a PET nonwoven fabric) available from Nitto Denko Corporation was used. The coating was formed on the PVDF porous layer of the RS-50. The application of the coating liquid was carried out using an applicator with a gap of 255 μm. Next, the coating was dried to form a separation functional layer. Thereby, a separation membrane of Example 1 was obtained.

(Examples 2 to 5)

Separation membranes of Examples 2 to 5 were obtained in the same manner as in Example 1, except that the metal organic frameworks shown in Table 3 were used instead of the MAF-Mg.

The TEVZIF used in Example 2 was produced by the following method. First, 0.1297 g (0.10 mmol) of γ-cyclodextrin, 0.2503 g (0.90 mmol) of lead(II) chloride, and 30 mL of ion exchanged water (IEW) were added into a 50 mL sample tube. Next, the obtained mixed solution was stirred for 1 hour using a hot stirrer set at 85°C. Subsequently, the mixed solution was cooled to a room temperature and filtered to remove a solid included in the mixed solution. The obtained filtrate was added into a 10OmL three-necked flask. Next, the filtrate was heated to 110°C under a nitrogen atmosphere while being stirred. Next, 30 mL of a mixed solution obtained by mixing cyclohexanol and triethylamine at 1/4 (v/v) was dropped gradually into the filtrate. After the dropping of the mixed solution was finished, the resultant mixture was refluxed at a condition of 110°C for three days, so that a reaction proceeded. The obtained reaction mixture was cooled to a room temperature and filtered to obtain a residue. The obtained residue was washed with IEW and air-dried at a room temperature. Thereby, white powder (TEVZIF) was obtained in an amount of 0.2810 g.

The JEDKIM used in Example 3 was produced by the following method. First, 0.6819 g (4.0 mmol) of copper(II) chloride dihydrate and 40 mL of IEW were added into a 100 mL beaker and stirred. Next, 0.3522 g (2.0 mmol) of L(+)-ascorbic acid was added, and the resultant mixture was stirred at a room temperature for 1 hour. Subsequently, the resultant mixed solution was filtered to remove a white solid therefrom. Next, the resultant filtrate was added into a 100 mL Erlenmeyer flask and stirred at a room temperature. To the filtrate, 0.3410 g (2.0 mmol) of copper(II) chloride dihydrate was added, and then 9 mL of an NaOH aqueous solution with a concentration of 1 mol/L was further added. The obtained reaction solution was left at rest at a room temperature for four weeks. Thereby, a blue solid was precipitated

in the reaction mixture. The reaction mixture was filtered to obtain a residue. The obtained residue was washed with IEW and air-dried at a room temperature. Thereby, blue-green powder (JEDKIM) was obtained in an amount of 0.2362 g.

The XUJKET (catena-[($\mu_2$-1H-Benzotriazol-1-ol-$N^2$,$N^3$)-($\mu_2$-1H-benzotriazol-1-olato-$N^2$,$N^3$)-silver(i)]) used in Example 4 was produced by the following method. First, 0.4247 g (2.5 mmol) of silver(I) nitrate, 0.3378 g (2.5 mmol) of 1-hydroxybenzotriazole (anhydrous (HOBt)), and 50 mL of IEW were added into a 100 mL crucible that was for hydrothermal synthesis and made of PTFE and stirred. An NaOH aqueous solution with a concentration of 3 mol/L was dropped gradually into the obtained mixed solution until the mixed solution had a pH of 3. Subsequently, this crucible was completely sealed with a stainless steel jacket and hydrothermal synthesis was allowed to proceed at 110°C for three days. Next, the reaction solution was cooled to a room temperature at a cooling rate of -6°C /hour. The obtained reaction mixture was filtered to obtain a residue. The obtained residue was washed with ethanol and air-dried at a room temperature. Thereby, gray powder (XUJKET) was obtained in an amount of 0.4307 g.

As the HKUST-1 in Example 5, Basolite (registered trademark) C300 available from BASF SE was used.

[Physical properties of metal organic framework]

On each of the metal organic frameworks used in Examples 1 to 5, the ratio R1 was determined by the method mentioned above. For the molecular simulations, Materials Studio available from Dassault Systemes and Gaussian09 available from Gaussian, Inc. were used. The crystal structure data of each of the metal organic frameworks was acquired from papers and known databases. On the metal organic framework (XUJKET) used in Example 4, a range in which the value $\Delta E1$ was a negative value failed to be found. That is, the potential energy $E_{MOF + H2O}$ of the composite composed of the metal organic framework and the water molecule(s) was never less than the total value of the potential energy $E_{MOF}$ of the metal organic framework S1 and the potential energy $E_{H2O}$ of the water molecule(s). Therefore, it was impossible to determine the ratio R1 on the metal organic framework (XUJKET) used in Example 4.

On each of the metal organic frameworks used in Examples 1 to 5, the ratio R2 was determined by the method mentioned above. The fillers used to determine the adsorption amounts Q1 and Q2 had a weight of 0.02 to 0.1 g. The pretreatment of the fillers was carried out for 6 hours under a vacuum atmosphere and a heat condition of 150°C. As the vapor adsorption amount measuring apparatus, BELSORP-maxII available from MicrotracBEL Corp. was used. In the measurements of the adsorption amounts, the adsorption of the gas (ethanol or water vapor) by each of the fillers was considered to have reached a state of equilibrium when a change in a pressure inside the measuring apparatus was 40 Pa or less for 500 seconds.

On each of the metal organic frameworks used in Examples 1 to 5, the void fraction and the largest cavity diameter were determined. The void fraction and the largest cavity diameter of each of the metal organic frameworks were determined by analyzing the crystal structure data of each of the metal organic frameworks using Zeo++.

On each of the metal organic frameworks used in Examples 1 to 5, the volume change ratios W1 and W2 were determined by the methods mentioned above. On the metal organic framework (XUJKET) used in Example 4, a range in which the value $\Delta E3$ was a negative value and a range in which the value $\Delta E4$ was a negative value failed to be found, thus it was impossible to determine the volume change ratios W1 and W2.

On the metal organic framework (TEVZIF) of Example 2, an actual measurement value of the volume change ratio of the crystal structure with respect to ethanol as well as an actual measurement value of the volume change ratio of the crystal structure with respect to water were determined. Specifically, the actual measurement value of the volume change ratio of the crystal structure with respect to ethanol was determined by the following method. First, 0.1 g of the filler composed of TEVZIF was physically mixed with 0.1 g of silicon (available from NACALAI TESQUE, INC., with a purity of 99.99% or more) for 5 minutes using an agate mortar. Next, the obtained mixture was dried under a vacuum atmosphere at 120°C for 6 hours to obtain a measurement sample 1.

Next, the measurement sample 1 was subject to an X-ray diffraction (XRD) measurement. For the XRD measurement, Smart Lab, an automated multipurpose X-ray diffractometer available from Rigaku Corporation, was used. As an X-ray beam, a CuK$\alpha$ ray (wavelength $\lambda$ = 1.54 Å) was used. When the measurement sample 1 was placed in the X-ray diffractometer, the measurement sample 1 was spread all over on a measuring stand in such a manner that a thin film thereof with a thickness of 0.5 mm was formed. Specific conditions of the XRD measurement were as follows.

- X-ray tube

  Tube: Cu
  Tube voltage: 45 kV
  Tube current: 200 mA

- Slits

Incident slit: 0.10 mm
Receiving slit 1: 0.20 mm
Receiving slit 2: 0.20 mm

- Measurement

Goniometer: wide-angle goniometer
Attachment: standard sample holder
Detector: D-TEX (none)
Sampling width: 0.0010°
Scanning speed: 5°/min
Scanning range: 5° to 30°
Scanning axis: 2 8/8

With regard to the result of the XRD measurement, a peak is adjusted based on an internal standard (silicon), so that a peak of a (101) plane, a peak of a (111) plane and a peak of a (200) plane of the metal organic framework included in the measurement sample 1 were determined. Next, from these peaks, a spacing of the (101) plane, a spacing of the (111) plane, and a spacing of the (200) plane were calculated using Bragg's formula ($2d\sin\theta = \eta\lambda$). Since TEVZIF is orthorhombic, a relation (IX) below holds between a spacing d and plane indices (h, k, l). Based on the relation (IX), lengths (a, b, c) of respective sides of a unit lattice of the metal organic framework included in the measurement sample 1 were calculated, and a volume $V_{before}$ of the unit lattice was also calculated. [Formula 3]

$$\frac{1}{d^2} = \frac{h^2}{a^2} + \frac{k^2}{b^2} + \frac{l^2}{c^2} \qquad (IX)$$

Next, in the state in which the measurement sample 1 was placed in the X-ray diffractometer, 200 μL of ethanol was dropped into the measurement sample 1 to obtain the measurement sample 2. In order to prevent the ethanol from evaporating from the measurement sample 2, the measurement sample 2 was covered with a Kapton film (type H with a thickness of 25 μm, available from Toray Industries, Inc.) and a periphery of the film was fixed with a cellophane tape. In the measurement sample 2, the metal organic framework was in contact with the ethanol for 10 minutes or more. Next, the measurement sample 2 was subject to the XRD measurement using the same method as that used for the measurement sample 1. Based on the obtained result, lengths (a, b, c) of respective sides of a unit lattice of the metal organic framework included in the measurement sample 2 as well as a volume $V_{after}$ of the unit lattice were calculated. Based on the volume $V_{after}$ and the volume $V_{before}$ obtained, the actual measurement value of the volume change ratio of the crystal structure with respect to ethanol was determined by formula (X) below.
[Formula 4]

$$\text{Actual measurement value} = \frac{V_{after} - V_{before}}{V_{before}} \times 100 \qquad (X)$$

The actual measurement value of the volume change ratio of the crystal structure with respect to water was determined by the same method as the one used for determining the actual measurement value of the volume change ratio of the crystal structure with respect to ethanol, except that water was used instead of the ethanol.

[Characteristics of separation membrane]

On each of the separation membranes of Examples 1 to 5, the flux F of the water permeating through the separation membrane and the separation factor α were measured by the following method. First, the separation membrane was placed in a metal cell, and the metal cell was sealed with an O-ring so that no leakage occurred. Next, 250 mL of a liquid mixture was injected into the metal cell in such a manner that the liquid mixture was in contact with a principal surface, on a side of the separation functional layer, of the separation membrane. The liquid mixture was composed substantially of ethanol and water. A concentration of the ethanol in the liquid mixture was 50 vol% when measured with a temperature of the liquid mixture at 20°C. Next, the metal cell was heated to 60°C in a water bath. The temperature of the liquid mixture in the metal cell was confirmed to be 60°C, and then a space, in the metal cell, that is adjacent to a principal surface, on a side of the porous support member, of the separation membrane was decompressed.

This space was decompressed in such a manner that a pressure in the space was lower than an atmospheric pressure in a measurement environment by 100 kPa. Thereby, a gaseous permeation fluid was obtained. The gaseous permeation fluid was cooled using -196°C liquid nitrogen to liquefy the permeation fluid. A composition of the liquid permeation fluid was analyzed using gas chromatography (GC-3200 available from GL Sciences Inc.). The flux of the water that had permeated through the separation membrane and the separation factor $\alpha$ of the separation membrane were calculated based on the composition of the permeation fluid, a weight of the permeation fluid, etc.

[Table 3]

| | Metal organic framework | | | | | | | | | Evaluation on separation membrane | | Thickness T of separation functional layer (μm) | T × F (μm•kg/$m^2$/hr) |
|---|---|---|---|---|---|---|---|---|---|---|---|---|---|
| | Type | Ratio R1 | Ratio R2 | Void fraction (%) | Largest cavity diameter (nm) | Volume change ratio W2 with respect to $H_2O$ (Sim: %) | Volume change ratio W1 with respect to EtOH (Sim: %) | Actual measurement value of volume change ratio with respect to $H_2O$ (Exp: %) | Actual measurement value of volume change ratio with respect to EtOH (Exp: %) | Flux F of water (kg/$m^2$/hr) | Separation factor α | | |
| Example 1 | MAF-Mg | 0.10 | 15.9 | 45.4 | 0.476 | 33.1 | 30.3 | - | - | 0.266 | 22.4 | 15.0 | 3.99 |
| Example 2 | TEVZIF | 0.10 | 9.1 | 45.6 | 0.836 | 16.0 | -6.0 | 10.0 | -2.3 | 0.159 | 82.0 | 12.0 | 1.91 |
| Example 3 | JEDKIM | 0.26 | 5.8 | 68.9 | 1.515 | 4.4 | -14.2 | - | - | 0.194 | 108.6 | 11.5 | 2.23 |
| Example 4 | XUJKET | - | 4.0 | 32.5 | 0.220 | - | - | - | - | 0.113 | 29.1 | 13.0 | 1.47 |
| Example 5 | HKUST-1 | 0.31 | 3.5 | 52.4 | 0.686 | -2.1 | -5.2 | - | - | 0.094 | 67.6 | 11.0 | 1.03 |

As shown in Table 3, on each of the separation membranes of Examples 1 to 3 including the metal organic framework that satisfied at least one of the requirements that the ratio R1 is less than 0.29 and that the ratio R2 was more than 4.0, the flux F of the water as well as the product of the flux F of the water and the thickness T of the separation functional layer resulted in values larger than those of the separation membranes of Examples 4 and 5. Furthermore, each of the separation membranes of Examples 1 to 3 had the separation factor $\alpha$ that was 20 or more, which was a practically sufficient value.

As shown in Table 3, on each of the separation membranes of Examples 2, 3, and 5 including the metal organic framework having the volume change ratio W1 that was 0% or less, the separation factor $\alpha$ resulted in a value larger than those of the separation membranes of Examples 1 and 4. As shown in Example 2, the volume change ratios W1 and W2 calculated by the molecular simulations were almost the same as the actual measurement values.

INDUSTRIAL APPLICABILITY

The separation membrane of the present embodiment is suitable for separating water from a liquid mixture containing an alcohol and water. Particularly, the separation membrane of the present embodiment is useful for refining bioethanol.

**Claims**

1. A separation membrane comprising a metal organic framework, wherein

    at least one of requirements (i) and (ii) below holds:

    (i) when the metal organic framework satisfies a condition (a) in which, when a number N1 of water molecules is/are inserted inside the metal organic framework, a potential energy of a composite composed of the metal organic framework and the water molecule(s) is lower than a total value of a potential energy of the metal organic framework in an isolated state before the water molecule(s) is/are inserted and a potential energy of the water molecule(s) in an isolated state, and a difference between the potential energy of the composite and the total value is maximum, and a condition (b) in which, when a number N2 of ethanol molecules is/are inserted inside the metal organic framework, a value obtained by subtracting a total value of a potential energy of the metal organic framework in an isolated state before the ethanol molecule(s) is/are inserted and a potential energy of the ethanol molecule(s) in an isolated state from a potential energy of a composite composed of the metal organic framework and the ethanol molecule(s) is minimum,

    a ratio R1 of the number N2 with respect to the number N1 is less than 0.29;
    (ii) a ratio R2 of an adsorption amount of water adsorbed by the metal organic framework under water vapor at 25°C and 3.2 kPa with respect to an adsorption amount of ethanol adsorbed by the metal organic framework under an ethanol atmosphere at 25°C and 7.4 kPa is more than 4.0.

2. The separation membrane according to claim 1, wherein the separation membrane is used for separating water from a liquid mixture containing an alcohol and water.

3. The separation membrane according to claim 1 or 2, wherein at least one of requirements that the ratio R1 is 0.14 or less and that the ratio R2 is 7.0 or more holds.

4. The separation membrane according to any one of claims 1 to 3, wherein the metal organic framework has a void fraction of 40% or more.

5. The separation membrane according to any one of claims 1 to 4, wherein the metal organic framework has a largest cavity diameter of 0.3 nm or more.

6. The separation membrane according to any one of claims 1 to 5, wherein the metal organic framework includes at least one selected from the group consisting of MAF-Mg, TEVZIF, and JEDKIM.

7. The separation membrane according to any one of claims 1 to 6, wherein a crystal structure of the metal organic framework has a volume change ratio, calculated by a molecular simulation, of 0% or less with respect to ethanol.

**8.** The separation membrane according to any one of claims 1 to 7, wherein
a crystal structure of the metal organic framework has a volume change ratio, calculated by a molecular simulation, of 0% or more with respect to water.

**9.** The separation membrane according to any one of claims 1 to 8, wherein

the separation membrane includes a matrix containing polyimide, and
the metal organic framework is dispersed in the matrix.

**10.** The separation membrane according to claim 9, wherein the polyimide includes a structural unit represented by formula (1) below:

[Chemical Formula 1]

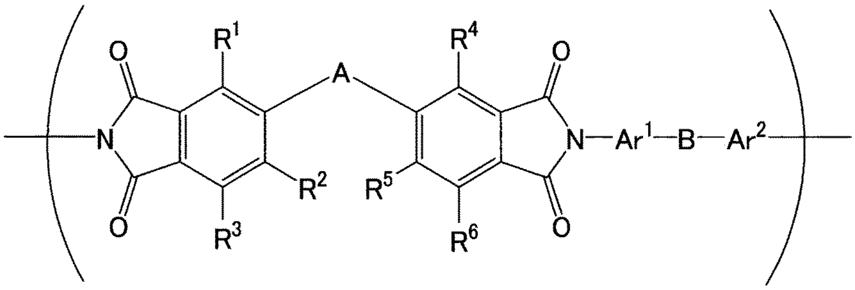

(1)

where A is a linking group having a solubility parameter, in accordance with a Fedors method, of more than 5.0 $(cal/cm^3)^{1/2}$; B is a linking group having a solubility parameter, in accordance with the Fedors method, of more than 8.56 $(cal/cm^3)^{1/2}$; $R^1$ to $R^6$ each are independently a hydrogen atom, a halogen atom, a hydroxyl group, a sulfonic group, an alkoxy group having 1 to 30 carbon atoms, or a hydrocarbon group having 1 to 30 carbon atoms; $Ar^1$ and $Ar^2$ each are a divalent aromatic group; and $Ar^1$ and $Ar^2$ each are represented by formula (2) below when $Ar^1$ and $Ar^2$ each are a phenylene group that may have a substituent;

[Chemical Formula 2]

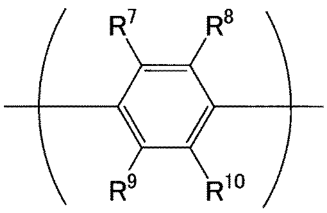

(2)

where $R^7$ to $R^{10}$ each are independently a hydrogen atom, a halogen atom, a hydroxyl group, a sulfonic group, an alkoxy group having 1 to 30 carbon atoms, or a hydrocarbon group having 1 to 30 carbon atoms.

**11.** The separation membrane according to any one of claims 1 to 10, wherein

when, in a state in which a liquid mixture composed of ethanol and water is in contact with one surface of the separation membrane, a space adjacent to an other surface of the separation membrane is decompressed, a flux of the water permeating through the separation membrane is 0.12 $kg/m^2/hr$ or more, and
a concentration of the ethanol in the liquid mixture is 50 vol% when measured with a temperature of the liquid mixture at 20°C, the liquid mixture in contact with the separation membrane has a temperature of 60°C, and the space is decompressed in such a manner that a pressure in the space is lower than an atmospheric pressure in a measurement environment by 100 kPa.

**12.** The separation membrane according to any one of claims 1 to 11, wherein

the separation membrane has a separation factor α of 20 or more for water with respect to ethanol,
in a state in which a liquid mixture composed of ethanol and water is in contact with one surface of the separation membrane, the separation factor α is measured by decompressing a space adjacent to an other surface of the separation membrane, and
a concentration of the ethanol in the liquid mixture is 50 vol% when measured with a temperature of the liquid mixture at 20°C, the liquid mixture in contact with the separation membrane has a temperature of 60°C, and the space is decompressed in such a manner that a pressure in the space is lower than an atmospheric pressure in a measurement environment by 100 kPa.

**13.** A separation membrane comprising a metal organic framework, wherein
a crystal structure of the metal organic framework has a volume change ratio, calculated by a molecular simulation, of 0% or less with respect to ethanol.

**14.** The separation membrane according to claim 13, wherein the crystal structure of the metal organic framework has a volume change ratio, calculated by the molecular simulation, of 0% or more with respect to water.

**15.** A metal organic framework used for separating water from a liquid mixture containing an alcohol and water, wherein

at least one of requirements (i) and (ii) below holds:

(i) when the metal organic framework satisfies a condition (a) in which, when a number N1 of water molecules is/are inserted inside the metal organic framework, a potential energy of a composite composed of the metal organic framework and the water molecule(s) is lower than a total value of a potential energy of the metal organic framework in an isolated state before the water molecule(s) is/are inserted and a potential energy of the water molecule(s) in an isolated state, and a difference between the potential energy of the composite and the total value is maximum, and a condition (b) in which, when a number N2 of ethanol molecules is/are inserted inside the metal organic framework, a value obtained by subtracting a total value of a potential energy of the metal organic framework in an isolated state before the ethanol molecule(s) is/are inserted and a potential energy of the ethanol molecule(s) in an isolated state from a potential energy of a composite composed of the metal organic framework and the ethanol molecule(s) is minimum,

a ratio R1 of the number N2 with respect to the number N1 is less than 0.29;
(ii) a ratio R2 of an adsorption amount of water adsorbed by the metal organic framework under water vapor at 25°C and 3.2 kPa with respect to an adsorption amount of ethanol adsorbed by the metal organic framework under an ethanol atmosphere at 25°C and 7.4 kPa is more than 4.0.

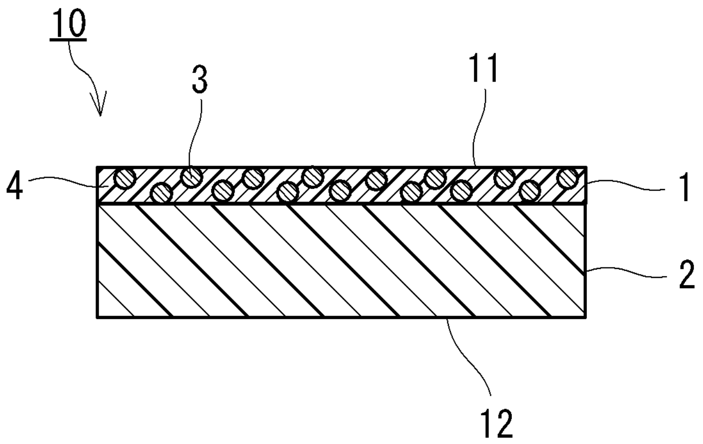


FIG.1

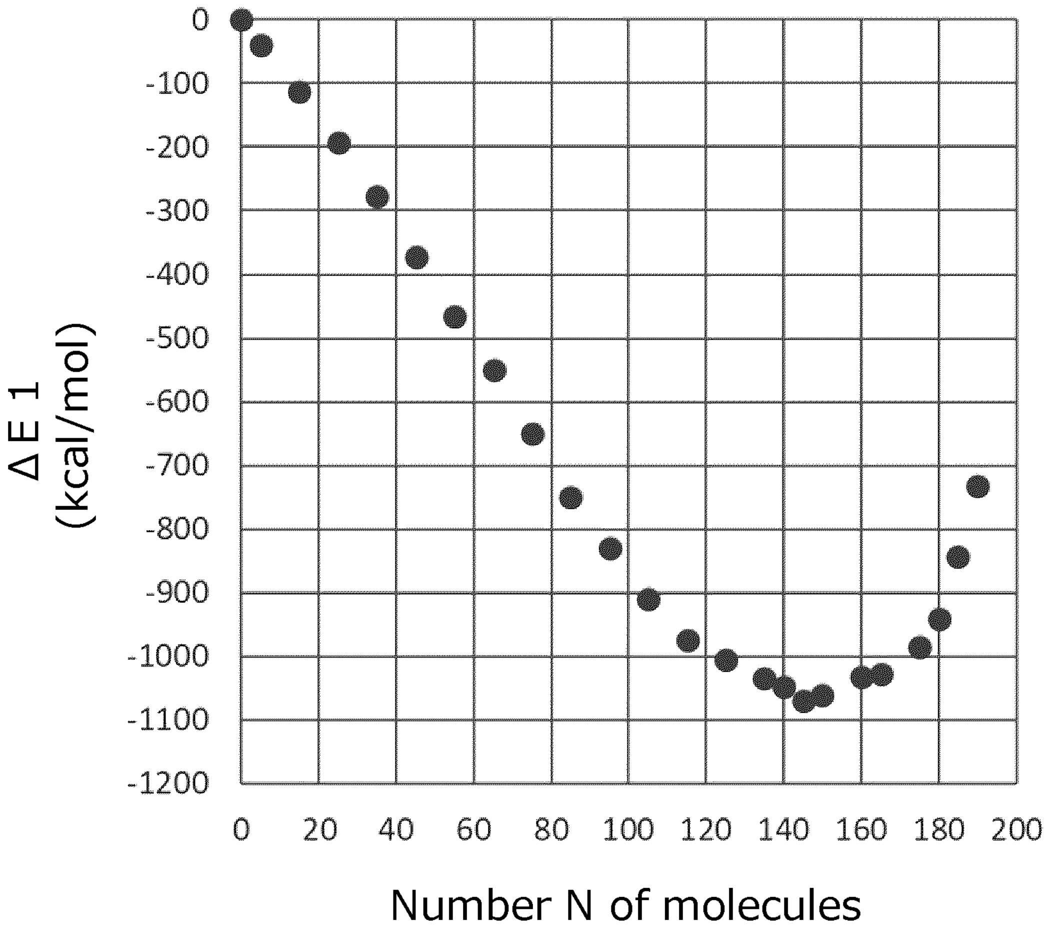
0
-100
-200
-300
-400
-500
-600
-700
-800
-900
-1000
-1100
-1200
ΔE 1
(kcal/mol)
0 20 40 60 80 100 120 140 160 180 200
Number N of molecules

FIG.2

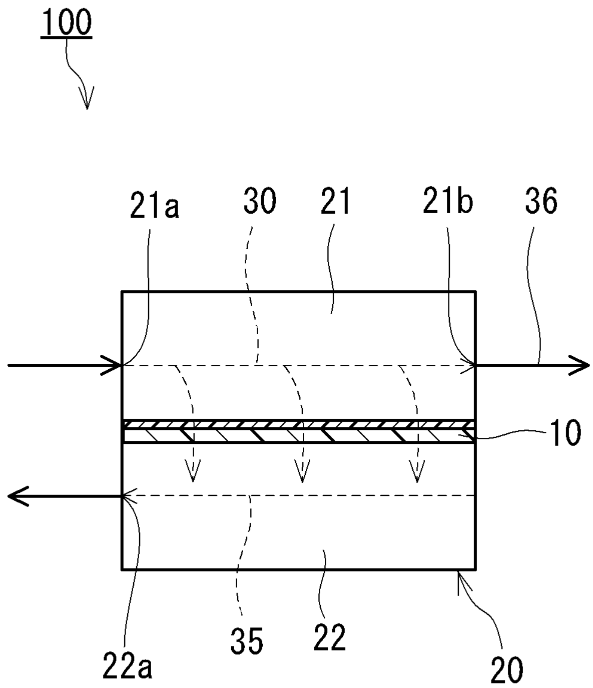


FIG.3

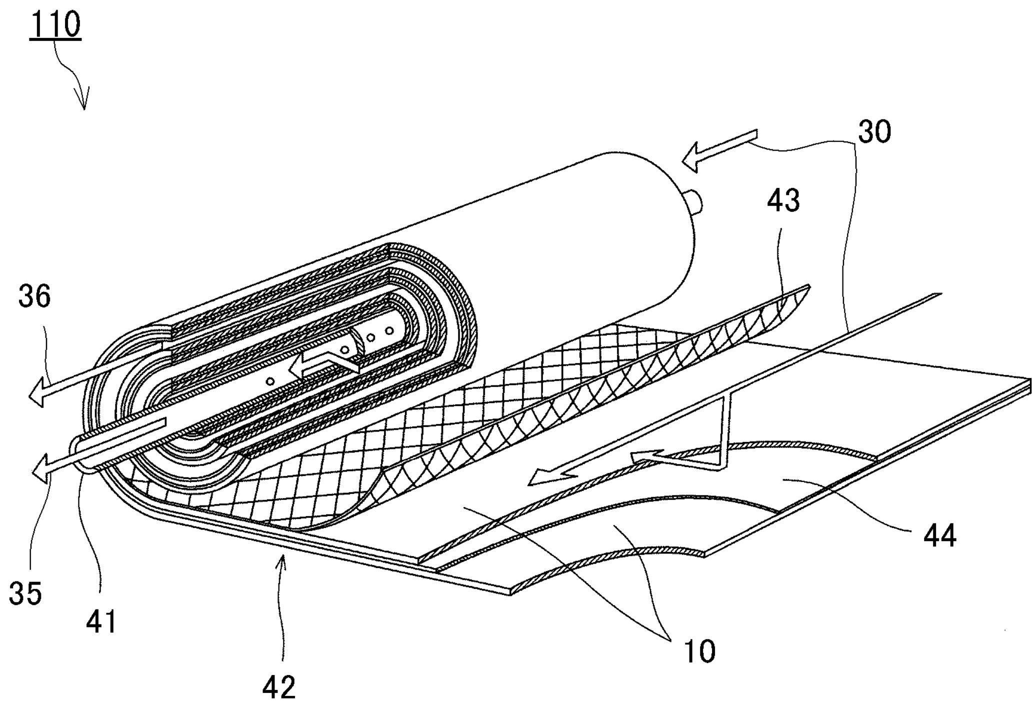


FIG.4

## INTERNATIONAL SEARCH REPORT

International application No. PCT/JP2021/002638

A. CLASSIFICATION OF SUBJECT MATTER
B01D 61/36(2006.01)i; B01D 69/00(2006.01)i; B01D 69/02(2006.01)i; B01D 71/06(2006.01)i; B01D 71/64(2006.01)i
FI: B01D69/02; B01D61/36; B01D69/00; B01D71/06; B01D71/64
According to International Patent Classification (IPC) or to both national classification and IPC

B. FIELDS SEARCHED

Minimum documentation searched (classification system followed by classification symbols)
B01D61/36; B01D69/00; B01D69/02; B01D71/06; B01D71/64

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2017-500204 A (SABIC GLOBAL TECHNOLOGIES B.V.) 05 January 2017 (2017-01-05) entire text, all drawings | 1-15 |
| A | WO 2012/159224 A1 (DALIAN INSTITUTE OF CHEMICAL PHYSICS, CHINESE ACADEMY OF SCIENCES) 29 November 2012 (2012-11-29) entire text, all drawings | 1-15 |
| A | JP 2014-523338 A (UOP LLC) 11 September 2014 (2014-09-11) entire text, all drawings | 1-15 |
| P, A | WO 2020/084996 A1 (NITTO DENKO CORP.) 30 April 2020 (2020-04-30) entire text, all drawings | 1-15 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 March 2021 (26.03.2021) | 06 April 2021 (06.04.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application no.
PCT/JP2021/002638

| Patent Documents referred in the Report | Publication Date | Patent Family | Publication Date |
|---|---|---|---|
| JP 2017-500204 A | 05 Jan. 2017 | US 2015/0101986 A1<br>entire text, all drawings<br>WO 2015/057429 A1<br>CN 105636671 A | |
| WO 2012/159224 A1 | 29 Nov. 2012 | US 2014/0212940 A1<br>entire text, all drawings<br>CN 103842052 A | |
| JP 2014-523338 A | 11 Sep. 2014 | US 2012/0322911 A1<br>entire text, all drawings<br>WO 2012/173776 A2<br>CA 2836127 A1<br>KR 10-2013-0137238 A<br>CN 103608094 A | |
| WO 2020/084996 A1 | 30 Apr. 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013528118 A


### Non-patent literature cited in the description


- **ROBERT F. FEDORS.** *Polymer Engineering and Science,* 1974, (14), 147-154